# EUROPEAN PATENT APPLICATION

(11) **EP 4 710 937 A1**
(43) Date of publication of application: **18.03.2026**
(21) Application number: 24807168.0
(22) Date of filing: 10.05.2024
(51) Int. Cl.: A61K 39/21, A61K 31/7088, A61K 35/76, A61K 39/39, A61K 48/00, A61P 35/02, A61P 37/04, C07K 14/15, C12N 15/48, C12N 15/63

(54) **PHARMACEUTICAL COMPOSITION**

(30) Priority: 12.05.2023 JP 2023079265
(71) Applicant: National University Corporation Kumamoto University, Kumamoto-shi, Kumamoto 860-8555 (JP); Eisai R&D Management Co., Ltd., Tokyo 112-8088 (JP)
(72) Inventor: MATSUOKA, Masao, Kumamoto-shi, Kumamoto 860-8555 (JP); YASUNAGA, Jun-ichirou, Kumamoto-shi, Kumamoto 860-8555 (JP); SUZUKI, Yuta, Tsukuba-shi, Ibaraki 300-2635 (JP); KUBARA, Kenji, Tsukuba-shi, Ibaraki 300-2635 (JP); MIYAZAKI, Takayuki, Tsukuba-shi, Ibaraki 300-2635 (JP)
(74) Representative: Hoffmann Eitle
(86) International application number: PCT/JP2024/017522
(87) International publication number: WO 2024/237216

(57) **Abstract**

[Problem] The purpose is to provide a novel pharmaceutical composition that can be used to induce an immune response to HTLV-1. [Solution] The pharmaceutical composition of the present disclosure comprises: human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier

## Description

### Technical Field

The present disclosure relates to a pharmaceutical composition.

### Background Art

Human T-cell leukemia virus type-I (HTLV-1) is a retrovirus known to cause the development of diseases such as adult T-cell leukemia-lymphoma (ATL) and HTLV-1-associated inflammatory diseases, e.g., HTLV-1-associated myelopathy (HAM) and HTLV-1-associated uveitis (HU). These HTLV-1-associated diseases develop in HTLV-1-infected individuals (carriers). Approximately 800,000 HTLV-1-infected individuals (carriers) reportedly reside in Japan. In addition, there are no subjective symptoms in most cases even when HTLV-1 infection occurs.

Since ATL is resistant to treatment and has a poor prognosis, preventing infection with HTLV-1 is regarded as important for preventing the onset of ATL. In addition, ATL patients who have undergone hematopoietic stem cell transplantation may experience a relapse of ATL after transplantation. For these reasons, in order to improve the prognosis for ATL treatment, it is also important to induce an immune response against HTLV-1. However, a pharmaceutical composition that is effective against HTLV-1 has yet to be established.

### Citation List

### Non Patent Literature

NPL 1: Cook, Lucy B. et al., "Revised Adult T-Cell Leukemia-Lymphoma International Consensus Meeting Report." Journal of Clinical Oncology: official journal of the American Society of Clinical Oncology vol. 37, 8 (2019): 677-687. doi:10.1200/JCO.18.00501

### Summary of Invention

### Technical Problem

Therefore, the purpose of the present disclosure is to provide a pharmaceutical composition that can be used to induce an immune response to HTLV-1.

### Solution to Problem

In order to accomplish this purpose, the pharmaceutical composition of the present disclosure comprises the following: human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

The pharmaceutical composition of the present disclosure comprises the following: nucleic acid that encodes human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

The pharmaceutical composition of the present disclosure comprises the following: a vector that contains nucleic acid that encodes human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

### Effect of the Invention

The pharmaceutical composition of the present disclosure can induce an immune response to HTLV-1.

### Brief Description of Drawings

[Fig. 1]Fig. 1 contains graphs that report results for the LIPS assay in Example 1.
[Fig. 2]Fig. 2 contains graphs that report results for the RT-qPCR in Example 1.
[Fig. 3]Fig. 3 contains photographs that report results for the proximity ligation assay in Example 1.
[Fig. 4]Fig. 4 contains photographs that show the results of the ELISpot assay in Example 1.
[Fig. 5]Fig. 5 contains photographs that show the results of the ELISpot assay in Example 1.
[Fig. 6]Fig. 6 is a graph that reports the results of the ELISpot assay in Example 2.

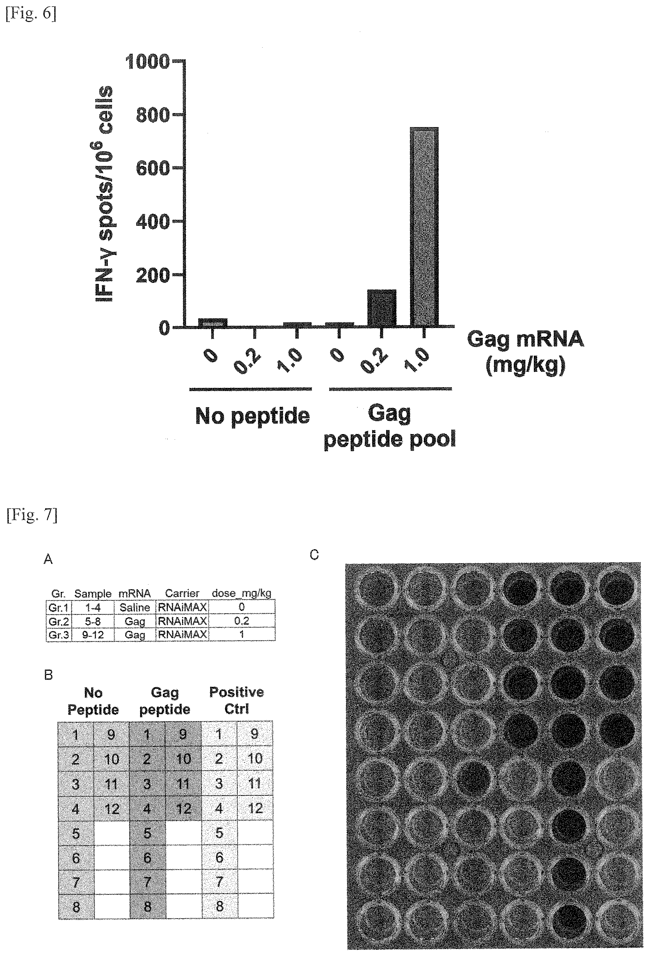

[Fig. 7]Fig. 7 contains photographs that report the results for the ELISpot assay in Example 2. The sample numbers in Fig. 7A correspond to the numbers (1 to 12) given in Fig. 7B. Samples 1 to 4 for No peptide, Gag peptide, and Positive Ctrl in Fig. 7B indicate samples where physiological saline was administered; samples 5 to 8 indicate samples where lipid-mRNA particles were administered at 0.2 mg/kg; and samples 9 to 12 indicate samples where lipid-mRNA particles were administered at 1 mg/kg. The results in Fig. 7C correspond to the respective sample positions indicated in Fig. 7B: the first and second rows from the left give the results for No peptide samples 1 to 12; the third and fourth rows from the left give the results for Gag peptide samples 1 to 12; and the fifth and sixth rows from the left give the results for the Positive Ctrl samples 1 to 12.

### Description of Embodiments

The present disclosure is described in the following using examples. In the description that follows, the descriptions of individual inventions can be cited by each other, unless specifically indicated otherwise.

### <Definition>

In the present Description, "protein" denotes a peptide polymer composed of unmodified amino acids (natural amino acids), modified amino acids, and/or artificial amino acids. The configuration of this polymer is, for example, straight-chain, branched, or cyclic. A protein can also be referred to as a peptide or a polypeptide.

In the present Description, a "retrovirus" is a type of RNA virus and denotes an RNA virus that has a reverse transcriptase. It is known that a retrovirus infects a host cell and propagates as follows. The retrovirus infects the host cell that is the infection target, double-stranded DNA is synthesized by reverse transcription of the viral RNA, and the viral DNA is incorporated as a provirus into the genomic DNA of the host cell. The host cell then produces viral RNA and viral proteins, a retroviral protease cleaves the immature viral proteins, and the mature viral proteins are assembled to form viral particles. The viral particles then bud from the surface of the host cell.

In the present Description, "HTLV-1" is a type of retrovirus and refers to human T-cell leukemia virus type-I. HTLV-1 is a virus known to cause diseases such as adult T-cell leukemia-lymphoma (ATL (or ATLL)), HTLV-1-associated myelopathy (HAM, TSP: tropical spastic paraparesis), and HTLV-1 uveitis (HU). HTLV-1 is a virus belonging to the Oncovirinae subfamily of the Retroviridae family. HTLV-1 has viral structural genes such as Gag, Pol, and Env. HTLV-1 also has regulatory genes such as Tax and Rex. HTLV-1 additionally has accessory genes such as p12, p13, p30, and HBZ. The proteins composed of the amino acid sequences registered at Genbank under the accession number AB513134.1 are an example for proviral HTLV-1.

In the present Description, "HTLV-1-associated diseases" is a general term for diseases caused by HTLV-1 infection. Examples of "HTLV-1-associated diseases" are 1) ATL, 2) HAM, and 3) HU.

In the present Description, "ATL" or "ATLL" refers to adult T-cell leukemia-lymphoma. It is known that in HTLV-1-infected individuals (carriers), HTLV-1 infects, inter alia, CD4+ T cells, resulting in the development of ATL by causing the infected cells to become cancerous.

In the present Description, "HAM" refers to HTLV-1-associated myelopathy (TSP: tropical spastic paraparesis).

In the present Description, "HU" refers to HTLV-1 uveitis or HTLV-1-associated uveitis.

In the present Description, "processing" refers to reactions whereby the translated polypeptide serving as a precursor is cleaved by, e.g., a protease, or is modified and is thereby processed into a more mature protein.

In the present Description, "Gag protein (also referred to as Gag in the following)" refers to a retroviral capsid precursor protein (unprocessed Gag protein). The Gag is known to play a role in, e.g., the assembly and budding of virus particles. The Gag is processed by retroviral protease. The Gag of HTLV-1 is processed by the protease into p15 (nucleocapsid protein), p19 (matrix protein), p24 (capsid protein), and so forth. An example of the Gag of HTLV-1 is the protein (SEQ ID NO: 1) composed of the amino acid sequence registered at Genbank under accession number AAA85841.1.
Amino acid sequence (SEQ ID NO: 1) of HTLV-1 Gag 
MGQIFSRSASPIPRPPRGLAAHHWLNFLQAAYRLEPGPSSYDFHQLKKFLKIALETPV
WICPINYSLLASLLPKGYPGRVNEILHILIQTQAQIPSRPAPPPPSSPTHDPPDSDPQIPPP
YVEPTAPQVLPVMHPHGAPPNHRPWQMKDLQAIKQEVSQAAPGSPQFMQTIRLAVQ
QFDPTAKDLQDLLQYLCSSLVASLHHQQLDSLISEAETRGITGYNPLAGPLRVQANN
PQQQGLRREYQQLWLAAFAALPGSAKDPSWASILQGLEEPYHAFVERLNIALDNGLP
EGTPKDPILRSLAYSNANKECQKLLQARGHTNSPLGDMLRACQTWTPKDKTKVLVV
QPKKPPPNQPCFRCGKAGHWSRDCTQPRPPPGPCPLCQDPTHWKRDCPRLKPTIPEPE
PEEDALLLDLPADIPHPKNSIGGEV
Base sequence (SEQ ID NO: 2) for Gag of HTLV-1

5'-atgggccaaatcttttcccgtagcgctagccctattccgcggccgccccgggggctggccgctcatcactggcttaacttcctccag

gcggcatatcgcctagaacccggtccctccagttacgatttccaccagttaaaaaaatttcttaaaatagctttagaaacaccagtctgga

tctgtcccattaactactccctcctagccagcctactcccaaaaggataccccggccgggtgaatgaaattttacacatactcatccaaac

ccaagcccagatcccgtcccgtcccgcgccaccgccgccgtcatcccccacccacgaccccccggattctgatccacaaatccccc

ctccctatgttgagcctacggccccccaagtccttccagtcatgcacccacatggtgcccctcccaaccatcgcccatggcaaatgaaa

gacctacaggccattaagcaagaagtctcccaagcagcccctgggagcccccagtttatgcagaccatccggcttgcggtgcagca

gtttgaccccactgccaaagacctccaagacctcctgcagtacctttgctcctccctcgtggcttccctccatcaccagcagctagatag

ccttatatcagaggccgaaacccgaggtattacaggttataaccccttagccggtcccctccgtgtccaagccaacaatccacaacaac

aaggattaaggcgagaataccagcaactctggctcgccgccttcgccgccctgccggggagtgccaaagacccttcctgggcctcta

tcctccaaggcctggaggagccttaccacgccttcgtagaacgcctcaacatagctcttgacaatgggctgccagaaggcacgccca

aagaccccatcttacgttccttagcctactccaatgcaaacaaagaatgccaaaaattactacaggcccgaggacacactaatagccct

ctaggagatatgttgcgggcttgtcagacctggacccccaaagacaaaaccaaagtgttagttgtccagcctaaaaaaccccccccaa

atcagccgtgcttccggtgcgggaaagcaggccactggagtcgggactgcactcagcctcgccccccccccgggccatgccccct

atgtcaagacccaactcactggaagcgagactgcccccgcctaaagcccactatcccagaaccagagccagaggaagatgccctcc

tattagacctccccgctgacatcccacacccaaaaaactccatagggggggaggtttaa-3'

In the present Description, "Tax protein (also referred to as Tax in the following)" refers to a transcription activator that activates both the retroviral LTR (long terminal repeat) and intracellular promoters. The Tax is known to play an important role in viral infectivity and malignant transformation of infected cells. An example of the Tax of HTLV-1 is the protein (SEQ ID NO: 3) composed of the amino acid sequence registered at Genbank under accession number P03409.2.
Amino acid sequence (SEQ ID NO: 3) of HTLV-1 Tax 
MAHFPGFGQSLLFGYPVYVFGDCVQGDWCPISGGLCSARLHRHALLATCPEHQITW

DPIDGRVIGSALQFLIPRLPSFPTQRTSKTLKVLTPPITHTTPNIPPSFLQAMRKYSPFRN

GYMEPTLGQHLPTLSFPDPGLRPQNLYTLWGGSVVCMYLYQLSPPITWPLLPHVIFC

HPGQLGAFLTNVPYKRIEELLYKISLTTGALIILPEDCLPTTLFQPARAPVTLTAWQNG
 
LLPFHSTLTTPGLIWTFTDGTPMISGPCPKDGQPSLVLQSSSFIFHKFQTKAYHPSFLLS

HGLIQYSSFHSLHLLFEEYTNIPISLLFNEKEADDNDHEPQISPGGLEPPSEKHFRETEV
Base sequence (SEQ ID NO: 4) for Tax of HTLV-1

5'-atggcccacttcccagggtttggacagagtcttcttttcggatacccagtctacgtgtttggagactgtgtacaaggcgactggtgcc
ccatctctgggggactatgttcggcccgcctacatcgtcacgccctactggccacctgtccagagcatcagatcacctgggaccccat
cgatggacgcgttatcggctcagctctacagttccttatccctcgactcccctccttccccacccagagaacctctaagaccctcaaggt
ccttaccccgccaatcactcatacaacccccaacattccaccctccttcctccaggccatgcgcaaatactcccccttccgaaatggata
catggaaccaacccttgggcagcacctcccaaccctgtcttttccagaccccggactccggccccaaaacctgtacaccctctgggga
ggctccgttgtctgcatgtacctctaccagctttccccccccatcacctggcccctcctgccccacgtgattttttgccaccccggccagc
tcggggccttcctcaccaatgttccctacaagcgaatagaagaactcctctataaaatttccctcaccacaggggccctaataattctacc
cgaagactgtttgcccaccacccttttccagcctgctagggcacccgtcacgctaacagcctggcaaaacggcctccttccgttccact
caaccctcaccactccaggccttatttggacatttaccgatggcacgcctatgatttccgggccctgccctaaagatggccagccatcttt
agtactacagtcctcctcctttatatttcacaaatttcaaaccaaggcctaccacccctcatttctactctcacacggcctcatacagtactct
tcctttcatagtttacatctcctgtttgaagaatacaccaacatccccatttctctactttttaacgaaaaagaggcagatgacaatgaccat
gagccccaaatatcccccgggggcttagagcctcccagtgaaaaacatttccgagaaacagaagtctga-3'

In the present Description, "HBZ protein (also referred to as HBZ in the following)" refers to a retroviral bZIP factor. The HBZ is known to contribute to control of viral RNA transcription. An example of HTLV-1 HBZ is the protein (SEQ ID NO: 5) composed of the amino acid sequence registered at Genbank under accession number BAE06226.1. In addition, with regard to the HBZ of HTLV-1, for example, a polynucleotide composed of the base sequence with the following SEQ ID NO: 6 (including the stop codon) is an example of a polynucleotide that encodes HBZ. In addition, the HBZ of HTLV-1, for example, may be a protein composed of the amino acid sequence given by SEQ ID NO: 7.
Amino acid sequence (SEQ ID NO: 5) of HTLV-1 HBZ MAASGLFRCLPVSCPEDLLVEELVDGLLSLEEELKDKEEEEAVLDGLLSLEEESRGRL 
RRGPPGEKAPPRGETHRDRQRRAEEKRKRKKEREKEEEKQTAEYLKRKEEEKARRR

RRAEKKAADVARRKQEEQERRERKWRQGAEKAKQHSARKEKMQELGIDGYTRQL

EGEVESLEAERRKLLQEKEDLMGEVNYWQGRLEAMWLQ
Base sequence (SEQ ID NO: 6) for HBZ of HTLV-1 
5'-
atggcggcctcagggctgtttcgatgcttgcctgtgtcatgcccggaggacctgctggtggaggaattggtggacgggctattatcctt
ggaggaagagttaaaggacaaggaggaggaggaagctgtgcttgacggtttgctatccttagaagaggaaagccgcggccggctg
cgacggggccctccaggggagaaagcgccacctcgcggggaaacgcatcgtgatcggcagcgacgggctgaggagaagagga
agcgaaaaaaagagcgggagaaagaggaggaaaagcagaccgctgagtatttgaaaaggaaggaagaggagaaggcacggcg
caggaggcgggcggagaagaaggccgctgacgtcgctaggaggaagcaggaagagcaggagcgccgtgagcgcaagtggag
acaaggggctgagaaggcgaaacagcatagtgctaggaaagaaaaaatgcaggagttggggattgatggctatactagacagttgg
aaggcgaggtggagtccttggaggctgaacggaggaagttgctgcaggagaaggaggatttgatgggagaggttaattattggcag
gggaggctggaggcgatgtggttgcaataa-3'
Amino acid sequence (SEQ ID NO: 7) of HTLV-1 HBZ 
MAASGLFRCLPVSCPEDLLVEELVDGLLSLEEELKDKEEEEAVLDGLLSLEEESRGRL

RRGPPGEKAPPRGETHRDRQRRAEEKRKRKKEREKEEEKQIAEYLKRKEEEKARRR

RRAEKKAADVARRKQEEQERRERKWRQGAEKAKQHSARKEKMQELGIDGYTRQL

EGEVESLEAERRKLLQEKEDLMGEVNYWQGRLEAMWLQ
Base sequence (SEQ ID NO: 8) for HBZ of HTLV-1

5'-atggcggcctcagggctgtttcgatgcttgcctgtgtcatgcccggaggacctgctggtggaggaattggtggacgggctattatc

cttggaggaagagttaaaggacaaggaggaggaggaagctgtgcttgacggtttgctatccttagaagaggaaagccgcggccggc

tgcgacggggccctccaggggagaaagcgccacctcgcggggaaacgcatcgtgatcggcagcgacgggccgaggagaagag

gaagcgaaaaaaagagcgggagaaagaggaggaaaagcagattgctgagtatttgaaaaggaaggaagaggagaaggcacggc

gcaggaggcgggcggagaagaaggccgctgacgtcgccaggaggaagcaggaagagcaggagcgccgtgagcgcaagtgga

gacaaggggctgagaaggcgaaacagcatagtgctaggaaagaaaaaatgcaggagttggggattgatggctatactagacagttg

gaaggcgaggtggagtccttggaggctgaacggaggaagttgctgcaggagaaggaggatttgatgggagaggttaattattggca

ggggaggctggaggcgatgtggttgcaataa-3'

In the present Description, "T cells" refer to cells that are a type of leukocyte, are classified as lymphocytes, and express a T cell receptor (TCR). Examples of T cells include CD4+ helper T cells and CD8+ cytotoxic T cells.

In the present Description, "antigenic" denotes the property wherein an antigen induces an immune response, e.g., antibody production or cellular immunity.

In the present Description, "immunogenic" denotes the property wherein an antigen induces an immune response, e.g., antibody production or cellular immunity. "Immunogenic fragment", also referred to as an immunogenic site, refers to an immune response-inducing fragment of a protein or polypeptide or an immune response-inducing truncated protein or polypeptide.

In the present Description, a "vaccine" denotes a composition that produces an immune response for the prevention and/or treatment of a disease or condition. The vaccine is thus a pharmaceutical product that contains an immunogenic drug and is designed for use to produce a specific defense and protective substances in humans or animals by vaccination.

In the present Description, a "pharmaceutically acceptable carrier" refers to a solvent and/or additive that can generally be used in pharmaceutical composition formulation technology. Pharmaceutically acceptable carriers are well known in the pertinent technical field. Pharmaceutically acceptable carriers include, for example, aqueous solutions or other solvents, such as water or buffered physiological saline; or solvents such as glycols, glycerol, and oils (e.g., olive oil); or organic esters for injection. The pharmaceutically acceptable carrier, for example, may contain a physiologically acceptable compound that acts to stabilize or enhance drug absorption. Examples of such physiologically acceptable compounds include carbohydrates such as glucose, sucrose, or dextran; antioxidants such as ascorbic acid or glutathione; chelating agents; low-molecular-weight proteins; or other stabilizers or excipients. Other components known to those skilled in the art, such as diluents, excipients, additives, and/or adjuvants, may be co-used. This pharmaceutically acceptable carrier is preferably almost or completely free of toxicity for organisms.

In the present Description, "adjuvant" denotes a compound that, when used in combination with a prescribed immunogen (antigen) in a formulation or composition, enhances, alters, or modifies the resulting immune response. Enhancement, alteration, or modification of the immune response refers, for example, to strengthening, increasing, or enhancing the specificity of the antibody response and/or the cellular immune response. Enhancement, alteration, or modification of the immune response may also refer to lowering, reducing, or suppressing a prescribed antigen-specific immune response.

In the present Description, "nucleic acid" denotes polymers of deoxyribonucleotides (DNA), ribonucleotides (RNA), and/or modified nucleotides. In the present Description, when "nucleic acid" is used in combination with a specific protein, the "nucleic acid" then denotes a nucleotide polymer that encodes the amino acid sequence of the protein. The nucleic acid may be, for example, genomic DNA, cDNA, mRNA, and so forth. The nucleic acid may be, for example, single-stranded or double-stranded. The nucleic acid may be interchangeably read as "polynucleotide" or "oligonucleotide". In the present disclosure, when the nucleic acid is mRNA, for example, uracil (u) can be read for thymine (t) in the examples of the polynucleotide base sequences given in the individual SEQ ID NOs provided in the following, and this description can be used for the base sequence of the mRNA. In addition, the nucleic acid, for example, may be modified (modified nucleic acid) or may not be modified. This modification can be exemplified by methylation, pseudouridylation, and thiation. In the present disclosure, when the nucleic acid is mRNA, for example, the uridine of the nucleic acid is preferably pseudouridylated, i.e., the uridine is preferably replaced with pseudouridine. This pseudouridylation may be carried out on a portion of the uridine or on all of the uridine.

**In** the present Description, a "vector" (expression vector) refers to a recombinant plasmid or virus that contains nucleic acid that is delivered to a host cell in vitro or in vivo.

**In** the present Description, "subject" refers to an animal or an animal-derived cell, tissue, or organ. "Subject" in particular is used as a term that includes humans. Animal refers to humans and non-human animals. Examples of non-human animals are mammals such as mice, rats, rabbits, dogs, cats, cows, horses, pigs, monkeys, dolphins, and sea lions.

**In** the present Description, "treatment" denotes reversing, alleviating, delaying the development of, or inhibiting the progression of the diseases described in the present Description.

**In the** present Description, "prevention" denotes reducing the potential for the onset of a disease or pathology; suppressing, delaying, or stopping the onset of a disease or pathology; suppressing, alleviating, delaying, or stopping the progression of a pathology; suppressing, reducing, delaying, or stopping the aggravation of a disease or pathology; or suppressing, delaying, or stopping the recurrence of a disease or pathology. This "prevention" may be, for example, the treatment of a subject (patient) who has developed the target disease, or the treatment of a model animal for the target disease.

**In** the present Description, "aggravation" denotes a worsening of the degree (severity) of a disease or pathology.

In the present Description, "isolated" denotes a state of being identified and separated, and/or a state of being recovered from components in the natural state. This "isolation" can be achieved, for example, by adopting at least one purification step.

The description in the present disclosure proceeds in the following using examples, but the present disclosure is not limited to or by the following examples and so forth and can be implemented using freely selected modifications. In the present disclosure, the individual descriptions can be cited by each other, unless specifically indicated otherwise. When the expression "-" or "to" is used in the present Description with reference to a range of values, it is used in the sense of including the numerical values or physical values before and after the expression. In the present Description, the expression "A and/or B" includes "A only", "B only", and "both A and B".

### <The pharmaceutical composition containing HTLV-1 Gag antigen>

In a certain aspect, the present disclosure relates to a pharmaceutical composition that can induce an immune response to HTLV-1 and in particular to the Gag of HTLV-1. The pharmaceutical composition of the present disclosure contains the following: human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

As a result of intensive investigations, the present inventors discovered that Gag, which is a structural protein of HTLV-1, is expressed in the cells of ATL patients and that cytotoxic T cells against Gag are present in patients who have not experienced a recurrence of ATL, and formulated the concept that it could be possible to effectively induce an immune response to HTLV-1 by using the Gag of HTLV-1 as an antigen. The present inventors also discovered that a cellular immune response by T cells can be induced by the use of a pharmaceutical composition in which an active ingredient is HTLV-1 Gag p15, p19, and p24, and thus came to establish the present disclosure. As a consequence, the pharmaceutical composition of the present disclosure, for example, is capable of inducing an immune response to HTLV-1.

The pharmaceutical composition of the present disclosure characteristically contains, as an active ingredient, HTLV-1 antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof (collectively also referred to in the following as the "immune antigen"), while its other features and conditions are not particularly limited. The pharmaceutical composition of the present disclosure - because it contains HTLV-1 antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof as an active ingredient - can induce an immune response to HTLV-1 when used as a pharmaceutical composition.

Gag p15 can be exemplified by the following proteins (a1), (a2), and (a3).
(a1) protein composed of the amino acid sequence of SEQ ID NO: 9 or 10
(a2) protein composed of an amino acid sequence in which one or a plurality of amino acids have been deleted, inserted, substituted, or added in the amino acid sequence of SEQ ID NO: 9 or 10
(a3) protein composed of an amino acid sequence having an at least 80% identity with the amino acid sequence of SEQ ID NO: 9 or 10

Amino acid sequence (SEQ ID NO: 9) of Gag p15 
VVQPKKPPPNQPCFRCGKAGHWSRDCTQPRPPPGPCPLCQDPTHWKRDCPRLKPTIP

EPEPEEDALLLDLPADIPHPKNLHRGGGLTSPPTLQQVLPNQDPASIL
Amino acid sequence (SEQ ID NO: 10) of Gag p15

VVQPKKPPPNQPCFRCGKAGHWSRDCTQPRPPPGPCPLCQDPTHWKRDCPRLKPTIP

EPEPEEDALLLDLPADIPHPKNSIGGEV

In the preceding (a1), the amino acid sequence given by SEQ ID NO: 9 or 10 is an amino acid sequence derived from HTLV-1. In addition, in the preceding (a1), the amino acid sequence given by SEQ ID NO: 9 is, for example, the sequence of endogenous mature Gag p15 that can be expressed in infected cells and is produced by the ribosome generating a frameshift. The amino acid sequence given by SEQ ID NO: 10 is, for example, a Gag p15 sequence that is produced when the ribosome does not generate a frameshift. For example, the amino acid sequence of SEQ ID NO: 10, which is predicted to be expressed in large amounts in vivo when used for a vaccine composition, is preferred for the amino acid sequence of (a1).

The "one or a plurality of" in the case of the protein (a2), for example, should be a range in which (a2) is a protein exhibiting immunogenicity with respect to HTLV-1. The "one or a plurality of" for (a2) is, for example, 1 to 31, 1 to 26, 1 to 25, 1 to 21, 1 to 15, 1 to 12, 1 to 10, 1 to 8, 1 to 5, 1 to 4, 1 to 3, 1 or 2, or 1 in the amino acid sequence of (a1). In the present disclosure, a numerical value range for a number of items, for example, indicates all of the positive integers belonging to that range. That is, for example, the phrase "1 to 5" indicates all of "1, 2, 3, 4, and 5" (this also applies in the following).

In the case of the protein (a2), the substitution is preferably a conservative substitution. A conservative substitution means that an amino acid residue is substituted by an amino acid residue having a similar side chain. Examples of conservative substitution are as follows: substitution between amino acid residues having a basic side chain, such as lysine, arginine, and histidine; substitution between amino acid residues having an acidic side chain, such as aspartic acid and glutamic acid; substitution between amino acid residues having an uncharged polar side chain, such as glycine, asparagine, glutamine, serine, threonine, tyrosine, and cysteine; substitution between amino acid residues having a nonpolar side chain, such as alanine, valine, leucine, isoleucine, proline, phenylalanine, methionine, and tryptophan; substitution between amino acid residues having a β-branched side chain, such as threonine, valine, and isoleucine; and substitution between amino acid residues having an aromatic side chain, such as tyrosine, phenylalanine, tryptophan, and histidine.

In the case of the protein (a3), the "identity", for example, should be a range in which (a3) is a peptide exhibiting immunogenicity with respect to HTLV-1. The "identity" of (a3) is, for example, at least 70%, at least 75%, at least 80%, at least 85%, at least 90%, at least 95%, at least 96%, at least 97%, at least 98%, or at least 99% relative to the amino acid sequence of (a1). The "identity" can be calculated, for example, by using default parameters in the BLAST homology algorithm (http://www.ncbi.nlm.nih.gov/BLAST/) of the National Center for Biotechnology Information (NCBI) (this also applies in the following).

Gag p19 can be exemplified by the following proteins (b1), (b2), and (b3).
(b1) protein composed of the amino acid sequence of SEQ ID NO: 11
(b2) protein composed of an amino acid sequence in which one or a plurality of amino acids have been deleted, inserted, substituted, or added in the amino acid sequence of SEQ ID NO: 11
(b3) protein composed of an amino acid sequence having an at least 80% identity with the amino acid sequence of SEQ ID NO: 11

Amino acid sequence (SEQ ID NO: 11) of Gag p19

MGQIFSRSASPIPRPPRGLAAHHWLNFLQAAYRLEPGPSSYDFHQLKKFLKIALETPV
WICPINYSLLASLLPKGYPGRVNEILHILIQTQAQIPSRPAPPPPSSPTHDPPDSDPQIPPP
YVEPTAPQVL

The "one or a plurality of" in the case of the protein (b2), for example, should be a range in which (b2) is a protein exhibiting immunogenicity with respect to HTLV-1. The "one or a plurality of" for (b2) is, for example, 1 to 38, 1 to 32, 1 to 25, 1 to 19, 1 to 12, 1 to 6, 1 to 5, 1 to 3, 1 or 2, or 1 in the amino acid sequence of (b1).

In the case of the protein (b3), the "identity", for example, should be a range in which (b3) is a peptide exhibiting immunogenicity with respect to HTLV-1. The "identity" of (b3) is, for example, at least 70%, at least 75%, at least 80%, at least 85%, at least 90%, at least 95%, at least 96%, at least 97%, at least 98%, or at least 99% relative to the amino acid sequence of (b1).

Gag p24 can be exemplified by the following proteins (c1), (c2), and (c3).
(c1) protein composed of the amino acid sequence of SEQ ID NO: 12
(c2) protein composed of an amino acid sequence in which one or a plurality of amino acids have been deleted, inserted, substituted, or added in the amino acid sequence of SEQ ID NO: 12
(c3) protein composed of an amino acid sequence having an at least 80% identity with the amino acid sequence of SEQ ID NO: 12

Amino acid sequence (SEQ ID NO: 12) of Gag p24

PVMHPHGAPPNHRPWQMKDLQAIKQEVSQAAPGSPQFMQTIRLAVQQFDPTAKDL

QDLLQYLCSSLVASLHHQQLDSLISEAETRGITGYNPLAGPLRVQANNPQQQGLRRE

YQQLWLAAFAALPGSAKDPSWASILQGLEEPYHAFVERLNIALDNGLPEGTPKDPIL

RSLAYSNANKECQKLLQARGHTNSPLGDMLRACQTWTPKDKTKVL

The "one or a plurality of" in the case of the protein (c2), for example, should be a range in which (c2) is a protein exhibiting immunogenicity with respect to HTLV-1. The "one or a plurality of" for (c2) is, for example, 1 to 64, 1 to 53, 1 to 42, 1 to 32, 1 to 21, 1 to 10, 1 to 8, 1 to 6, 1 to 4, 1 or 2, or 1 in the amino acid sequence of (c1).

In the case of the protein (c3), the "identity", for example, should be a range in which (c3) is a peptide exhibiting immunogenicity with respect to HTLV-1. The "identity" of (c3) is, for example, at least 70%, at least 75%, at least 80%, at least 85%, at least 90%, at least 95%, at least 96%, at least 97%, at least 98%, or at least 99% relative to the amino acid sequence of (c1).

In the present disclosure, the immunogenic fragments of p15, p19, and p24, for example, should be a range in which the corresponding protein is a protein exhibiting immunogenicity with respect to HTLV-1. The immunogenic fragments of p15 and p19 may be, for example, a freely selected protein fragment of these proteins having a length of at least 5, 10, 20, 30, 40, 50, 60, 70, 80, 90, 100, or more than 100 amino acids.

The immunogenic fragment of p19 can be exemplified by the amino acid sequence at positions 2 to 59 (p19-1 in the examples provided below), the amino acid sequence at positions 52 to 109 (p19-2 in the examples provided below), and the amino acid sequence at positions 102 to 130 (p19-3 in the examples provided below) of SEQ ID NO: 1, and is preferably the amino acid sequence at positions 2 to 59 (p19-1 in the examples provided below) or the amino acid sequence at positions 52 to 109 (p19-2 in the examples provided below) of SEQ ID NO: 1.

The immunogenic fragment of p19 may be, for example, a peptide that encodes an amino acid sequence having an identity of at least 70%, at least 75%, at least 80%, at least 85%, at least 90%, at least 95%, at least 96%, at least 97%, at least 98%, or at least 99% relative to the base sequence of any of the amino acid sequence at positions 2 to 59, the amino acid sequence at positions 52 to 109, and the amino acid sequence at positions 102 to 130 of SEQ ID NO: 1.

The pharmaceutical composition of the present disclosure, for example, may contain any one of the following, or may contain a plurality of the following, or may contain all of the following: Gag p15 or an immunogenic fragment thereof, Gag p19 or an immunogenic fragment thereof, and Gag p24 or an immunogenic fragment thereof. The pharmaceutical composition of the present disclosure, for example, preferably contains the Gag p15 or an immunogenic fragment thereof, plus the Gag p19 or an immunogenic fragment thereof, plus the Gag p24 or an immunogenic fragment thereof. By having such a construction, the pharmaceutical composition of the present disclosure, for example, can efficiently induce an immune response to HTLV-1.

Gag p15, Gag p19, and Gag p24 are expressed via processing of the unprocessed Gag protein. The pharmaceutical composition of the present disclosure may therefore contain, for example, the unprocessed Gag protein or an immunogenic fragment thereof and so forth, for Gag p15, Gag p19, and/or Gag p24. As a consequence, the Gag unprocessed protein or an immunogenic fragment thereof, for example, can also refer to precursors or precursor proteins for Gag p15 or an immunogenic fragment thereof, Gag p19 or an immunogenic fragment thereof, and/or Gag p24 or an immunogenic fragment thereof.

The unprocessed Gag can be exemplified by the following proteins (d1), (d2), and (d3).
(d1) protein composed of the amino acid sequence of SEQ ID NO: 1
(d2) protein composed of an amino acid sequence in which one or a plurality of amino acids have been deleted, inserted, substituted, or added in the amino acid sequence of SEQ ID NO: 1
(d3) protein composed of an amino acid sequence having an at least 80% identity with the amino acid sequence of SEQ ID NO: 1

The "one or a plurality of" in the case of the protein (d2), for example, should be a range in which (d2) is a protein exhibiting immunogenicity with respect to HTLV-1. The "one or a plurality of" for (d2) is, for example, 1 to 128, 1 to 107, 1 to 85, 1 to 64, 1 to 42, 1 to 21, 1 to 14, 1 to 12, 1 to 8, 1 to 4, 1 or 2, or 1 in the amino acid sequence of (d1).

In the case of the protein (d3), the "identity", for example, should be a range in which (d3) is a peptide exhibiting immunogenicity with respect to HTLV-1. The "identity" of (d3) is, for example, at least 70%, at least 75%, at least 80%, at least 85%, at least 90%, at least 95%, at least 96%, at least 97%, at least 98%, or at least 99% relative to the amino acid sequence of (d1).

The unprocessed Gag is preferably a protein that contains at least one of the amino acid sequence at positions 2 to 59 (p19-1 in the examples provided below), the amino acid sequence at positions 52 to 109 (p19-2 in the examples provided below), and the amino acid sequence at positions 102 to 130 (p19-3 in the examples provided below) of SEQ ID NO: 1, with the region outside said amino acid sequence being composed of the amino acid sequence in SEQ ID NO: 1 that is outside said amino acid sequence or being composed of an amino acid sequence having an identity of at least 80%, at least 85%, at least 90%, at least 95%, at least 96%, at least 97%, at least 98%, or at least 99% relative to the amino acid sequence in SEQ ID NO: 1 that is outside said amino acid sequence. The unprocessed Gag is more preferably a protein that contains at least one of the amino acid sequence at positions 2 to 59 (p19-1 in the examples provided below) and the amino acid sequence at positions 52 to 109 (p19-2 in the examples provided below) of SEQ ID NO: 1, with the region outside said amino acid sequence being composed of the amino acid sequence in SEQ ID NO: 1 that is outside said amino acid sequence or being composed of an amino acid sequence having an identity of at least 80%, at least 85%, at least 90%, at least 95%, at least 96%, at least 97%, at least 98%, or at least 99% relative to the amino acid sequence in SEQ ID NO: 1 that is outside said amino acid sequence.

In the present disclosure, the immunogenic fragment of unprocessed Gag, for example, should be a range in which the corresponding protein is a protein that exhibits immunogenicity with respect to HTLV-1. The immunogenic fragment of unprocessed Gag may be, for example, a freely selected protein fragment of this protein having a length of at least 5, 10, 20, 30, 40, 50, 60, 70, 80, 90, 100, or more than 100 amino acids.

The pharmaceutical composition of the present disclosure may further comprise, for example, the Tax protein of HTLV-1 or an immunogenic fragment thereof, and/or the HBZ protein or an immunogenic fragment thereof. By additionally containing, for example, Tax or an immunogenic fragment thereof, and/or HBZ protein or an immunogenic fragment thereof, the pharmaceutical composition of the present disclosure can more efficiently induce an immune response against HTLV-1.

Tax can be exemplified by the following proteins (e1), (e2), and (e3).
(e1) protein composed of the amino acid sequence of SEQ ID NO: 3
(e2) protein composed of an amino acid sequence in which one or a plurality of amino acids have been deleted, inserted, substituted, or added in the amino acid sequence of SEQ ID NO: 3
(e3) protein composed of an amino acid sequence having an at least 80% identity with the amino acid sequence of SEQ ID NO: 3

The "one or a plurality of" in the case of the protein (e2), for example, should be a range in which (e2) is a protein exhibiting immunogenicity with respect to HTLV-1. The "one or a plurality of" for (e2) is, for example, 1 to 105, 1 to 88, 1 to 70, 1 to 52, 1 to 35, 1 to 17, 1 to 14, 1 to 10, 1 to 7, 1 to 3, 1 or 2, or 1 in the amino acid sequence of (e1).

### In the case of the protein (e3), the "identity", for example, should be a range in which (e3) is a peptide exhibiting immunogenicity with respect to HTLV-1. The "identity" of (e3) is, for example, at least 70%, at least 75%, at least 80%, at least 85%, at least 90%, at least 95%, at least 96%, at least 97%, at least 98%, or at least 99% relative to the amino acid sequence of (e1).

HBZ can be exemplified by the following proteins (f1), (f2), and (f3).
(f1) protein composed of the amino acid sequence of SEQ ID NO: 5 or 7
(f2) protein composed of an amino acid sequence in which one or a plurality of amino acids have been deleted, inserted, substituted, or added in the amino acid sequence of SEQ ID NO: 5 or 7
(f3) protein composed of an amino acid sequence having an at least 80% identity with the amino acid sequence of SEQ ID NO: 5 or 7

The "one or a plurality of" in the case of the protein (f2), for example, should be a range in which (f2) is a protein exhibiting immunogenicity with respect to HTLV-1. The "one or a plurality of" for (f2) is, for example, 1 to 62, 1 to 52, 1 to 41, 1 to 31, 1 to 20, 1 to 10, 1 to 8, 1 to 6, 1 to 4, or 1 or 2 in the amino acid sequence of (f1).

In the case of the protein (f3), the "identity", for example, should be a range in which (f3) is a peptide exhibiting immunogenicity with respect to HTLV-1. The "identity" of (f3) is, for example, at least 70%, at least 75%, at least 80%, at least 85%, at least 90%, at least 95%, at least 96%, at least 97%, at least 98%, or at least 99% relative to the amino acid sequence of (f1).

In the present disclosure, an immunogenic fragment of the Tax or HBZ, for example, should be a range in which the corresponding protein is a protein exhibiting immunogenicity with respect to HTLV-1. An immunogenic fragment of the Tax or HBZ may be, for example, a freely selected protein fragment of this protein having a length of at least 5, 10, 20, 30, 40, 50, 60, 70, 80, 90, 100, or more than 100 amino acids.

The pharmaceutical composition of the present disclosure, for example, can induce cellular immunity, in particular cytotoxic T cell-mediated cellular immunity, to HTLV-1 upon its administration to a subject. As a consequence, the pharmaceutical composition of the present disclosure can also be referred to as a vaccine, a vaccine composition, or a vaccine formulation.

The pharmaceutical composition of the present disclosure, for example, may be a vaccine for use in the prevention of the onset of ATL or the aggravation of ATL or the recurrence of ATL.

The pharmaceutical composition of the present disclosure contains a pharmaceutically acceptable carrier. In some embodiments, this pharmaceutically acceptable carrier can be exemplified by suspending agents, solubilizing agents, stabilizers, isotonicity agents, preservatives, adsorption inhibitors, surfactants, diluents, media, pH adjusters, soothing agents, buffers, sulfur-containing reducing agents, antioxidants, and so forth, in order to administer the active ingredient, and is added as appropriate within a range that does not impair the effects of the present disclosure.

The suspending agent is not particularly limited and can be exemplified by methyl cellulose, polysorbate 80, hydroxyethyl cellulose, gum arabic, tragacanth powder, sodium carboxymethyl cellulose, and polyoxyethylene sorbitan monolaurate.

The solution auxiliary agent is not particularly limited and can be exemplified by polyoxyethylene hydrogenated castor oil, polysorbate 80, nicotinamide, polyoxyethylene sorbitan monolaurate, macrogol, and ethyl esters of castor oil fatty acids.

The stabilizer is not particularly limited and can be exemplified by dextran 40, methyl cellulose, gelatin, sodium sulfite, and sodium metasulfate.

The isotonicity agent is not particularly limited and can be exemplified by D-mannitol and sorbitol.

The preservative is not particularly limited and can be exemplified by methyl para-hydroxybenzoate, ethyl para-hydroxybenzoate, sorbic acid, phenol, cresol, and chlorocresol.

The adsorption inhibitor is not particularly limited can be exemplified by human serum albumin, lecithin, dextran, ethylene oxide-propylene oxide copolymer, hydroxypropyl cellulose, methyl cellulose, hydrogenated castor oil, and polyethylene glycol.

The sulfur-containing reducing agent is not particularly limited and can be exemplified by sulfur-containing reducing agents that contain a sulfhydryl group, such as N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and thioalkanoic acids having 1 to 7 carbon atoms.

The antioxidant is not particularly limited and can be exemplified by erythorbic acid, dibutylhydroxytoluene, butylhydroxyanisole, α-tocopherol, tocopherol acetate, L-ascorbic acid and salts thereof, L-ascorbyl palmitate, L-ascorbyl stearate, sodium hydrogen sulfite, sodium sulfite, triamyl gallate, and propyl gallate, or chelating agents such as sodium ethylenediaminetetraacetate (EDTA), sodium pyrophosphate, and sodium metaphosphate.

Components that are generally added may also be added as appropriate to the pharmaceutical composition of the present disclosure, e.g., inorganic salts such as sodium chloride, potassium chloride, calcium chloride, sodium phosphate, potassium phosphate, and sodium bicarbonate; organic salts such as sodium citrate, potassium citrate, and sodium acetate; sugars such as glucose; and so forth.

The pharmaceutical composition of the present disclosure may also contain an adjuvant (immunostimulant) and so forth. The adjuvant can be exemplified by toll-like receptor stimulants such as CpG oligonucleotide (CpG ODN) and lipopolysaccharide (LPS); aluminum salts such as aluminum hydroxide, aluminum phosphate, and aluminum chloride; interferons such as interferon (IFN-γ); inflammatory cytokines such as TNF-α; interleukins such as IL-1, IL-2, IL-3, IL-4, IL-12, and IL-13; growth factors such as granulocytemacrophage (GM) colony-stimulating factor (GM-CSF) and granulocyte colony-stimulating factor (G-CSF); cytokines such as Flt3 ligand; B7-1; B7-2; chemokines; squalene-containing adjuvants such as squalane or squalene; complete or incomplete Freund's adjuvant; or mixtures of the preceding. An example of LPS is MPL (monophosphoryl lipid A). Examples of squalene-containing adjuvants are AddaVax (trademark) and MF-59 (registered trademark), which are oil-in-water emulsions of squalene.

The pharmaceutical composition of the present disclosure, for example, may be used in vitro or may be used in vivo. The pharmaceutical composition of the present disclosure, for example, may also be used as a reagent employed in research or may be used as a pharmaceutical product. In the former case, the pharmaceutical composition of the present disclosure may also be referred to as a test reagent or test kit.

The target for administration of the pharmaceutical composition of the present disclosure is not particularly limited. For use of the pharmaceutical composition of the present disclosure in vivo, those examples already provided above can be adopted as examples of the subject (subject of the administration). When the pharmaceutical composition of the present disclosure is used in vitro, the target for administration can be, for example, cells, tissue, an organ, and so forth, and the cells can be, for example, cells acquired from a living organism or cultured cells, while the tissue or organ can be, for example, tissue (living tissue) or an organ acquired from a living organism. The cells can be, for example, immune cells such as T cells, B cells, NK cells, dendritic cells, and so forth.

For the use of the pharmaceutical composition of the present disclosure in vivo, the recipient subject may be a healthy individual who is not infected with HTLV-1, an individual for whom a potential for infection with HTLV-1 exists, or a patient infected with HTLV-1. In addition, the recipient subject may be a patient who is infected with HTLV-1 and has not developed ATL, or who has developed ATL, or who is in remission from ATL. The recipient subject is preferably a subject who has become infected with HTLV-1 and for whom the prevention of ATL onset or ATL recurrence is desired. The recipient subject may be a patient who has received a bone marrow transplant or hematopoietic stem cell transplant after developing ATL.

The use conditions (administration conditions) for the pharmaceutical composition of the present disclosure are not particularly limited, and, for example, the form of administration, timing of administration, dose, and so forth can be established as appropriate in accordance with the type of active ingredient in the pharmaceutical composition, the type of subject of the administration, and so forth.

The method of administration of the pharmaceutical composition of the present disclosure can be exemplified by intracerebral administration, intrathecal administration, intramuscular administration, subcutaneous administration, intravenous administration, and so forth; however, for example, intramuscular administration or subcutaneous administration is preferred because this enables a safe and stable administration regardless of the skill of the individual performing the administration.

The dose of the pharmaceutical composition of the present disclosure is the amount that can induce an immune response in the recipient subject, i.e., is the effective dose. The dose, for example, can be established as appropriate depending on the age, weight, symptoms, and so forth of the recipient subject.

The number of administrations of the pharmaceutical composition of the present disclosure is one or a plurality of times. The plurality of times is, for example, two, three, four, five, or more times. The number of administrations may be appropriately determined while confirming the prophylactic effect on the subject of the administration. In the case of administration a plurality of times, the administration interval can be appropriately determined while confirming the prophylactic effect on the subject of the administration, and can be exemplified by once a day, once a week, once every two weeks, once a month, once every three months, once every six months, and so forth.

The pharmaceutical composition of the present disclosure can prevent or lessen at least one HTLV-1 infection-associated symptom in the subject of the administration. As a consequence, the pharmaceutical composition of the present disclosure, because it can induce an immune response to HTLV-1, can be used for the prevention and/or treatment of HTLV-1-associated diseases. An example of a symptom of HTLV-1 infection is the onset of ATL. Symptom prevention, for example, can be evaluated subjectively or objectively, and specific examples include self-assessment by the recipient subject; evaluation by a physician; quality of life (QOL) evaluation; and evaluation of the onset of ATL or a delay in the progression of ATL symptoms, or an evaluation of the reduction in the severity of ATL symptoms. The objective evaluation may be an evaluation using an animal or an evaluation by a human.

Each of the proteins for the pharmaceutical composition of the present disclosure can be synthesized by genetic engineering methods and, as a specific example, can be produced using the transformants described below. This protein may be produced, for example, by in vitro synthesis.

### <The nucleic acid-containing pharmaceutical composition>

In another aspect, the present disclosure relates to a pharmaceutical composition that can induce an immune response to HTLV-1 and in particular to HTLV-1 Gag. The pharmaceutical composition (the nucleic acid-containing pharmaceutical composition) of the present disclosure comprises: a nucleic acid that encodes human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

The nucleic acid-containing pharmaceutical composition of the present disclosure is characterized in that it contains, as an active ingredient, a nucleic acid that encodes HTLV-1 antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof, with there being no particular limitation on its other features and conditions. The nucleic acid-containing pharmaceutical composition of the present disclosure - because it contains, as an active ingredient, a nucleic acid that encodes HTLV-1 antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof - can induce an immune response to HTLV-1 when used as a pharmaceutical composition. The description of the pharmaceutical composition containing the Gag antigen of HTLV-1 can be applied to the nucleic acid-containing pharmaceutical composition of the present disclosure, unless specifically indicated otherwise.

The nucleic acid that encodes Gag p15 can be exemplified by polynucleotides selected from the group consisting of the following (A1) to (A6) and (A7):
(A1) a polynucleotide composed of the base sequence of SEQ ID NO: 13 or 14;
(A2) a polynucleotide composed of a base sequence in which one or a plurality of bases have been deleted, inserted, substituted, and/or added in a base sequence of (A1);
(A3) a polynucleotide composed of a base sequence having an at least 80% identity with either base sequence of (A1);
(A4) a polynucleotide composed of a base sequence that is complementary to a polynucleotide that hybridizes under stringent conditions with a polynucleotide composed of either base sequence of (A1);
(A5) a polynucleotide that encodes a protein composed of the amino acid sequence of SEQ ID NO: 9 or 10;
(A6) a polynucleotide that encodes a protein composed of an amino acid sequence in which one or a plurality of amino acids have been deleted, inserted, substituted, and/or added in an amino acid sequence of (A5); and
(A7) a polynucleotide that encodes a protein composed of an amino acid sequence having an at least 80% identity with an amino acid sequence of (A5).

The base sequences with SEQ ID NO: 13 or 14 of (A1) are given below. The base sequences with SEQ ID NO: 13 or 14 are the coding sequences for the Gag p15 composed of the amino acid sequence with SEQ ID NO: 9 or 10. A polynucleotide (A1) with SEQ ID NO: 13 or 14 can be obtained, for example, from HTLV-1.
Polynucleotide (SEQ ID NO: 13) of (A1) 5'-gttgtccagcctaaaaaaccccccccaaatcagccgtgcttccggtgcgggaaagcaggccactggagtcgggactgcactcag 
cctcgccccccccccgggccatgccccctatgtcaagacccaactcactggaagcgagactgcccccgcctaaagcccactatccc

agaaccagagccagaggaagatgccctcctattagacctccccgctgacatcccacacccaaaaaacctccatagggggggaggtt

taacctccccccccacattacagcaagtccttcctaaccaagacccagcatctattctg-3'
Polynucleotide (SEQ ID NO: 14) of (A1)

5'-
gttgtccagcctaaaaaaccccccccaaatcagccgtgcttccggtgcgggaaagcaggccactggagtcgggactgcactcagcc
tcgccccccccccgggccatgccccctatgtcaagacccaactcactggaagcgagactgcccccgcctaaagcccactatcccag
aaccagagccagaggaagatgccctcctattagacctccccgctgacatcccacacccaaaaaactccatagggggggaggtt-3'

For (A2), the "one or a plurality of", for example, should be a range in which the protein encoded by the polynucleotide of (A2) exhibits immunogenicity with respect to HTLV-1. The "one or a plurality of" for (A2) is, for example, 1 to 94, 1 to 78, 1 to 76, 1 to 63, 1 to 51, 1 to 47, 1 to 31, 1 to 25, 1 to 15, 1 to 12, 1 to 10, 1 to 9, 1 to 7, 1 to 6, 1 to 5, 1 or 2, or 1 in a base sequence of (A1).

For (A3), the "identity", for example, should be a range in which the protein encoded by the polynucleotide of (A3) exhibits immunogenicity with respect to HTLV-1. The "identity" of (A3) is, for example, at least 70%, at least 75%, at least 80%, at least 85%, at least 90%, at least 95%, at least 96%, at least 97%, at least 98%, or at least 99% relative to a base sequence of (A1).

For (A4), the "polynucleotide that hybridizes", for example, is a polynucleotide that is fully or partially complementary to a polynucleotide of (A1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted.

For (A4), the "stringent conditions" may be, for example, any of low stringent conditions, medium stringent conditions, or high stringent conditions. The "low stringent conditions" are, for example, 5×SSC, 5×Denhardt's solution, 0.5% SDS, 50% formamide, and 32°C. The "medium stringent conditions" are, for example, 5×SSC, 5×Denhardt's solution, 0.5% SDS, 50% formamide, and 42°C. The "high stringent conditions" are, for example, 5×SSC, 5×Denhardt's solution, 0.5% SDS, 50% formamide, and 50°C. The individual skilled in the art can establish the degree of stringency, for example, by the appropriate selection of conditions such as temperature, salt concentration, probe concentration and length, ionic strength, and time. The conditions described in, for example, "Molecular Cloning: A Laboratory Manual 2nd Ed.", edited by Sambrook et al. [Cold Spring Harbor Laboratory Press (1989)], can also be adopted for the "stringent conditions".

The polynucleotide of (A5) can be established, for example, by conversion to the corresponding codons based on the amino acid sequence of SEQ ID NO: 9 or 10.

For (A6), the "one or a plurality of", for example, should be a range in which the protein encoded by the polynucleotide of (A6) exhibits immunogenicity with respect to HTLV-1. The "one or a plurality of" for (A6) is, for example, 1 to 31, 1 to 26, 1 to 25, 1 to 21, 1 to 15, 1 to 12, 1 to 10, 1 to 8, 1 to 5, 1 to 4, 1 to 3, 1 or 2, or 1 in the amino acid sequence of (A5).

For (A7), the "identity", for example, should be a range in which the protein encoded by the polynucleotide of (A7) exhibits immunogenicity with respect to HTLV-1. The "identity" of (A7) is, for example, at least 70%, at least 75%, at least 80%, at least 85%, at least 90%, at least 95%, at least 96%, at least 97%, at least 98%, or at least 99% relative to the amino acid sequence of (A5).

The nucleic acid that encodes Gag p19 can be exemplified by polynucleotides selected from the group consisting of the following (B1) to (B6) and (B7):
(B1) a polynucleotide composed of the base sequence with SEQ ID NO: 15;
(B2) a polynucleotide composed of a base sequence in which one or a plurality of bases have been deleted, inserted, substituted, and/or added in the base sequence of (B1);
(B3) a polynucleotide composed of a base sequence having an at least 80% identity with any base sequence of (B1);
(B4) a polynucleotide composed of a base sequence that is complementary to a polynucleotide that hybridizes under stringent conditions with a polynucleotide composed of any base sequence of (B1);
(B5) a polynucleotide that encodes a protein composed of the amino acid sequence of SEQ ID NO: 11;
(B6) a polynucleotide that encodes a protein composed of an amino acid sequence in which one or a plurality of amino acids have been deleted, inserted, substituted, and/or added in the amino acid sequence of (B5); and
(B7) a polynucleotide that encodes a protein composed of an amino acid sequence having an at least 80% identity with the amino acid sequence of (B5).

The base sequence with SEQ ID NO: 15 of (B1) is given below. The base sequence with SEQ ID NO: 15 is the coding sequence for Gag p19 composed of the amino acid sequence with SEQ ID NO: 11. The polynucleotide (B1) with SEQ ID NO: 15 can be obtained, for example, from HTLV-1.
Polynucleotide (SEQ ID NO: 15) of (B1)

5'-ggccaaatcttttcccgtagcgctagccctattccgcggccgccccgggggctggccgctcatcactggcttaacttcctccaggc

ggcatatcgcctagaacccggtccctccagttacgatttccaccagttaaaaaaatttcttaaaatagctttagaaacaccagtctggatct

gtcccattaactactccctcctagccagcctactcccaaaaggataccccggccgggtgaatgaaattttacacatactcatccaaaccc

aagcccagatcccgtcccgtcccgcgccaccgccgccgtcatcccccacccacgaccccccggattctgatccacaaatcccccctc

cctatgttgagcctacggccccccaagtcctt-3'

For (B2), the "one or a plurality of", for example, should be a range in which the protein encoded by the polynucleotide of (B2) exhibits immunogenicity with respect to HTLV-1. The "one or a plurality of" for (B2) is, for example, 1 to 116, 1 to 96, 1 to 77, 1 to 58, 1 to 38, 1 to 19, 1 to 15, 1 to 11, 1 to 7, 1 to 3, 1 or 2, or 1 in the base sequence of (B1).

For (B3), the "identity", for example, should be a range in which the protein encoded by the polynucleotide of (B3) exhibits immunogenicity with respect to HTLV-1. The "identity" of (B3) is, for example, at least 70%, at least 75%, at least 80%, at least 85%, at least 90%, at least 95%, at least 96%, at least 97%, at least 98%, or at least 99% relative to the base sequence of (B1).

For (B4), the "polynucleotide that hybridizes", for example, is a polynucleotide that is fully or partially complementary to the polynucleotide of (B1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted. The description of the conditions in (A4) above can be adopted for the stringent conditions for (B4).

The polynucleotide of (B5) can be established, for example, by conversion to the corresponding codons based on the amino acid sequence of SEQ ID NO: 11.

For (B6), the "one or a plurality of", for example, should be a range in which the protein encoded by the polynucleotide of (B6) exhibits immunogenicity with respect to HTLV-1. The "one or a plurality of" for (B6) is, for example, 1 to 38, 1 to 32, 1 to 25, 1 to 19, 1 to 12, 1 to 6, 1 to 5, 1 to 3, 1 or 2, or 1 in the amino acid sequence of (B5).

For (B7), the "identity", for example, should be a range in which the protein encoded by the polynucleotide of (B7) exhibits immunogenicity with respect to HTLV-1. The "identity" of (B7) is, for example, at least 70%, at least 75%, at least 80%, at least 85%, at least 90%, at least 95%, at least 96%, at least 97%, at least 98%, or at least 99% relative to the amino acid sequence of (B5).

The nucleic acid that encodes Gag p24 can be exemplified by polynucleotides selected from the group consisting of the following (C1) to (C6) and (C7):
(C1) a polynucleotide composed of the base sequence with SEQ ID NO: 16;
(C2) a polynucleotide composed of a base sequence in which one or a plurality of bases have been deleted, inserted, substituted, and/or added in the base sequence of (C1);
(C3) a polynucleotide composed of a base sequence having an at least 80% identity with any base sequence of (C1);
(C4) a polynucleotide composed of a base sequence that is complementary to a polynucleotide that hybridizes under stringent conditions with a polynucleotide composed of any base sequence of (C1);
(C5) a polynucleotide that encodes a protein composed of the amino acid sequence of SEQ ID NO: 12;
(C6) a polynucleotide that encodes a protein composed of an amino acid sequence in which one or a plurality of amino acids have been deleted, inserted, substituted, and/or added in the amino acid sequence of (C5); and
(C7) a polynucleotide that encodes a protein composed of an amino acid sequence having an at least 80% identity with the amino acid sequence of (C5).

The base sequence with SEQ ID NO: 16 of (C1) is given below. The base sequence with SEQ ID NO: 16 is the coding sequence for Gag p24 composed of the amino acid sequence with SEQ ID NO: 12. The polynucleotide (C1) with SEQ ID NO: 16 can be obtained, for example, from HTLV-1.
Polynucleotide (SEQ ID NO: 16) of (C1)

5'-ccagtcatgcacccacatggtgcccctcccaaccatcgcccatggcaaatgaaagacctacaggccattaagcaagaagtctccc

aagcagcccctgggagcccccagtttatgcagaccatccggcttgcggtgcagcagtttgaccccactgccaaagacctccaagacc

tcctgcagtacctttgctcctccctcgtggcttccctccatcaccagcagctagatagccttatatcagaggccgaaacccgaggtattac

aggttataaccccttagccggtcccctccgtgtccaagccaacaatccacaacaacaaggattaaggcgagaataccagcaactctgg

ctcgccgccttcgccgccctgccggggagtgccaaagacccttcctgggcctctatcctccaaggcctggaggagccttaccacgcc

ttcgtagaacgcctcaacatagctcttgacaatgggctgccagaaggcacgcccaaagaccccatcttacgttccttagcctactccaat

gcaaacaaagaatgccaaaaattactacaggcccgaggacacactaatagccctctaggagatatgttgcgggcttgtcagacctgg

acccccaaagacaaaaccaaagtgtta-3'

For (C2), the "one or a plurality of", for example, should be a range in which the protein encoded by the polynucleotide of (C2) exhibits immunogenicity with respect to HTLV-1. The "one or a plurality of" for (C2) is, for example, 1 to 192, 1 to 160, 1 to 128, 1 to 96, 1 to 64, 1 to 32, 1 to 25, 1 to 19, 1 to 12, 1 to 6, or 1 or 2 in the base sequence of (C1).

For (C3), the "identity", for example, should be a range in which the protein encoded by the polynucleotide of (C3) exhibits immunogenicity with respect to HTLV-1. The "identity" of (C3) is, for example, at least 70%, at least 75%, at least 80%, at least 85%, at least 90%, at least 95%, at least 96%, at least 97%, at least 98%, or at least 99% relative to the base sequence of (C1).

For (C4), the "polynucleotide that hybridizes", for example, is a polynucleotide that is fully or partially complementary to the polynucleotide of (C1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted. The description of the conditions in (A4) above can be adopted for the stringent conditions for (C4).

The polynucleotide of (C5) can be established, for example, by conversion to the corresponding codons based on the amino acid sequence of SEQ ID NO: 12.

For (C6), the "one or a plurality of", for example, should be a range in which the protein encoded by the polynucleotide of (C6) exhibits immunogenicity with respect to HTLV-1. The "one or a plurality of" for (C6) is, for example, 1 to 64, 1 to 53, 1 to 42, 1 to 32, 1 to 21, 1 to 10, 1 to 8, 1 to 6, 1 to 4, 1 or 2, or 1 in the amino acid sequence of (C5).

For (C7), the "identity", for example, should be a range in which the protein encoded by the polynucleotide of (C7) exhibits immunogenicity with respect to HTLV-1. The "identity" of (C7) is, for example, at least 70%, at least 75%, at least 80%, at least 85%, at least 90%, at least 95%, at least 96%, at least 97%, at least 98%, or at least 99% relative to the amino acid sequence of (C5).

In the present disclosure, the nucleic acid for an immunogenic fragment of p15, p19, or p24, for example, should be a range in which the protein encoded by this polynucleotide exhibits immunogenicity with respect to HTLV-1.

The nucleic acid-containing pharmaceutical composition of the present disclosure, for example, may contain a nucleic acid that encodes any one of the following, or may contain nucleic acid that encodes a plurality of the following, or may contain a nucleic acid that encodes all of the following: Gag p15 or an immunogenic fragment thereof, Gag p19 or an immunogenic fragment thereof, and Gag p24 or an immunogenic fragment thereof. The nucleic acid-containing pharmaceutical composition of the present disclosure, for example, preferably contains a nucleic acid that encodes Gag p15 or an immunogenic fragment thereof, plus a nucleic acid that encodes Gag p19 or an immunogenic fragment thereof, plus a nucleic acid that encodes Gag p24 or an immunogenic fragment thereof. By having such a constitution, the nucleic acid-containing pharmaceutical composition of the present disclosure, for example, can efficiently induce an immune response to HTLV-1.

The nucleic acid-containing pharmaceutical composition of the present disclosure may contain, for the Gag p15, Gag p19, and/or Gag p24, a nucleic acid that encodes unprocessed Gag protein or an immunogenic fragment thereof and so forth.

The nucleic acid encoding the unprocessed Gag can be exemplified by polynucleotides selected from the group consisting of the following (D1) to (D6) and (D7):
(D1) a polynucleotide composed of the base sequence with SEQ ID NO: 2;
(D2) a polynucleotide composed of a base sequence in which one or a plurality of bases have been deleted, inserted, substituted, and/or added in the base sequence of (D1);
(D3) a polynucleotide composed of a base sequence having an at least 80% identity with any base sequence of (D1);
(D4) a polynucleotide composed of a base sequence that is complementary to a polynucleotide that hybridizes under stringent conditions with a polynucleotide composed of any base sequence of (D1);
(D5) a polynucleotide that encodes a protein composed of the amino acid sequence of SEQ ID NO: 1;
(D6) a polynucleotide that encodes a protein composed of an amino acid sequence in which one or a plurality of amino acids have been deleted, inserted, substituted, and/or added in the amino acid sequence of (D5); and
(D7) a polynucleotide that encodes a protein composed of an amino acid sequence having an at least 80% identity with the amino acid sequence of (D5).

The base sequence with SEQ ID NO: 2 of (D1) is given below. The base sequence with SEQ ID NO: 2 is the coding sequence for unprocessed Gag composed of the amino acid sequence with SEQ ID NO: 1. Polynucleotide (D1) with SEQ ID NO: 2 can be obtained, for example, from HTLV-1.
Polynucleotide (SEQ ID NO: 2) of (D1)

5'-atgggccaaatcttttcccgtagcgctagccctattccgcggccgccccgggggctggccgctcatcactggcttaacttcctccag

gcggcatatcgcctagaacccggtccctccagttacgatttccaccagttaaaaaaatttcttaaaatagctttagaaacaccagtctgga

tctgtcccattaactactccctcctagccagcctactcccaaaaggataccccggccgggtgaatgaaattttacacatactcatccaaac

ccaagcccagatcccgtcccgtcccgcgccaccgccgccgtcatcccccacccacgaccccccggattctgatccacaaatccccc

ctccctatgttgagcctacggccccccaagtccttccagtcatgcacccacatggtgcccctcccaaccatcgcccatggcaaatgaaa

gacctacaggccattaagcaagaagtctcccaagcagcccctgggagcccccagtttatgcagaccatccggcttgcggtgcagca

gtttgaccccactgccaaagacctccaagacctcctgcagtacctttgctcctccctcgtggcttccctccatcaccagcagctagatag

ccttatatcagaggccgaaacccgaggtattacaggttataaccccttagccggtcccctccgtgtccaagccaacaatccacaacaac

aaggattaaggcgagaataccagcaactctggctcgccgccttcgccgccctgccggggagtgccaaagacccttcctgggcctcta

tcctccaaggcctggaggagccttaccacgccttcgtagaacgcctcaacatagctcttgacaatgggctgccagaaggcacgccca

aagaccccatcttacgttccttagcctactccaatgcaaacaaagaatgccaaaaattactacaggcccgaggacacactaatagccct

ctaggagatatgttgcgggcttgtcagacctggacccccaaagacaaaaccaaagtgttagttgtccagcctaaaaaaccccccccaa

atcagccgtgcttccggtgcgggaaagcaggccactggagtcgggactgcactcagcctcgccccccccccgggccatgccccct

atgtcaagacccaactcactggaagcgagactgcccccgcctaaagcccactatcccagaaccagagccagaggaagatgccctcc

tattagacctccccgctgacatcccacacccaaaaaactccatagggggggaggtttaa-3'

For (D2), the "one or a plurality of", for example, should be a range in which the protein encoded by the polynucleotide of (D2) exhibits immunogenicity with respect to HTLV-1. The "one or a plurality of" for (D2) is, for example, 1 to 387, 1 to 322, 1 to 258, 1 to 192, 1 to 129, 1 to 64, 1 to 51, 1 to 42, 1 to 38, 1 to 25, 1 to 12, 1 to 6, 1 to 3, 1 or 2, or 1 in the base sequence of (D1).

For (D3), the "identity", for example, should be a range in which the protein encoded by the polynucleotide of (D3) exhibits immunogenicity with respect to HTLV-1. The "identity" of (D3) is, for example, at least 70%, at least 75%, at least 80%, at least 85%, at least 90%, at least 95%, at least 96%, at least 97%, at least 98%, or at least 99% relative to the base sequence of (D1).

For (D4), the "polynucleotide that hybridizes", for example, is a polynucleotide that is fully or partially complementary to the polynucleotide of (D1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted. The description of the conditions in (A4) above can be adopted for these stringent conditions for (D4).

The polynucleotide of (D5) can be established, for example, by conversion to the corresponding codons based on the amino acid sequence of SEQ ID NO: 1.

For (D6), the "one or a plurality of", for example, should be a range in which the protein encoded by the polynucleotide of (D6) exhibits immunogenicity with respect to HTLV-1. The "one or a plurality of" for (D6) is, for example, 1 to 128, 1 to 107, 1 to 85, 1 to 64, 1 to 42, 1 to 21, 1 to 14, 1 to 12, 1 to 8, 1 to 4, 1 or 2, or 1 in the amino acid sequence of (D5).

For (D7), the "identity", for example, should be a range in which the protein encoded by the polynucleotide of (D7) exhibits immunogenicity with respect to HTL V-1. The "identity" of (D7) is, for example, at least 70%, at least 75%, at least 80%, at least 85%, at least 90%, at least 95%, at least 96%, at least 97%, at least 98%, or at least 99% relative to the amino acid sequence of (D5).

In the present disclosure, the nucleic acid for an immunogenic fragment of unprocessed Gag, for example, should be in a range in which the protein encoded by this polynucleotide exhibits immunogenicity with respect to HTLV-1.

The nucleic acid-containing pharmaceutical composition of the present disclosure may additionally contain a nucleic acid that encodes the Tax protein of HTLV-1 or an immunogenic fragment thereof and/or the HBZ protein or an immunogenic fragment thereof. The nucleic acid-containing pharmaceutical composition of the present disclosure, for example, can more efficiently induce an immune response to HTLV-1 by additionally containing a nucleic acid that encodes Tax or an immunogenic fragment thereof and/or a nucleic acid that encodes the HBZ protein or an immunogenic fragment thereof.

The nucleic acid that encodes Tax can be exemplified by polynucleotides selected from the group consisting of the following (E1) to (E6) and (E7):
(E1) a polynucleotide composed of the base sequence with SEQ ID NO: 4;
(E2) a polynucleotide composed of a base sequence in which one or a plurality of bases have been deleted, inserted, substituted, and/or added in the base sequence of (E1);
(E3) a polynucleotide composed of a base sequence having an at least 80% identity with any base sequence of (E1);
(E4) a polynucleotide composed of a base sequence that is complementary to a polynucleotide that hybridizes under stringent conditions with a polynucleotide composed of any base sequence of (E1);
(E5) a polynucleotide that encodes a protein composed of the amino acid sequence of SEQ ID NO: 3;
(E6) a polynucleotide that encodes a protein composed of an amino acid sequence in which one or a plurality of amino acids have been deleted, inserted, substituted, and/or added in the amino acid sequence of (E5); and
(E7) a polynucleotide that encodes a protein composed of an amino acid sequence having an at least 80% identity with the amino acid sequence of (E5).

The base sequence with SEQ ID NO: 4 of (E1) is given below. The base sequence with SEQ ID NO: 4 is the coding sequence for the Tax composed of the amino acid sequence with SEQ ID NO: 3. The polynucleotide (E1) with SEQ ID NO: 4 can be obtained, for example, from HTLV-1.
Polynucleotide (SEQ ID NO: 4) of (E1)

5'-atggcccacttcccagggtttggacagagtcttcttttcggatacccagtctacgtgtttggagactgtgtacaaggcgactggtgcc

ccatctctgggggactatgttcggcccgcctacatcgtcacgccctactggccacctgtccagagcatcagatcacctgggaccccat

cgatggacgcgttatcggctcagctctacagttccttatccctcgactcccctccttccccacccagagaacctctaagaccctcaaggt

ccttaccccgccaatcactcatacaacccccaacattccaccctccttcctccaggccatgcgcaaatactcccccttccgaaatggata

catggaaccaacccttgggcagcacctcccaaccctgtcttttccagaccccggactccggccccaaaacctgtacaccctctgggga

ggctccgttgtctgcatgtacctctaccagctttccccccccatcacctggcccctcctgccccacgtgattttttgccaccccggccagc

tcggggccttcctcaccaatgttccctacaagcgaatagaagaactcctctataaaatttccctcaccacaggggccctaataattctacc

cgaagactgtttgcccaccacccttttccagcctgctagggcacccgtcacgctaacagcctggcaaaacggcctccttccgttccact

caaccctcaccactccaggccttatttggacatttaccgatggcacgcctatgatttccgggccctgccctaaagatggccagccatcttt

agtactacagtcctcctcctttatatttcacaaatttcaaaccaaggcctaccacccctcatttctactctcacacggcctcatacagtactct

tcctttcatagtttacatctcctgtttgaagaatacaccaacatccccatttctctactttttaacgaaaaagaggcagatgacaatgaccat

gagccccaaatatcccccgggggcttagagcctcccagtgaaaaacatttccgagaaacagaagtctga-3'

For (E2), the "one or a plurality of", for example, should be a range in which the protein encoded by the polynucleotide of (E2) exhibits immunogenicity with respect to HTLV-1. The "one or a plurality of" for (E2) is, for example, 1 to 318, 1 to 265, 1 to 212, 1 to 159, 1 to 106, 1 to 53, 1 to 42, 1 to 31, 1 to 21, 1 to 10, 1 to 6, 1 or 2, or 1 in the base sequence of (E1).

For (E3), the "identity", for example, should be a range in which the protein encoded by the polynucleotide of (E3) exhibits immunogenicity with respect to HTLV-1. The "identity" of (E3) is, for example, at least 70%, at least 75%, at least 80%, at least 85%, at least 90%, at least 95%, at least 96%, at least 97%, at least 98%, or at least 99% relative to the base sequence of (E1).

For (E4), the "polynucleotide that hybridizes", for example, is a polynucleotide that is fully or partially complementary to the polynucleotide of (E1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted. The description of the conditions in (A4) above can be adopted for these stringent conditions for (E4).

The polynucleotide of (E5) can be established, for example, by conversion to the corresponding codons based on the amino acid sequence of SEQ ID NO: 3.

For (E6), the "one or a plurality of", for example, should be a range in which the protein encoded by the polynucleotide of (E6) exhibits immunogenicity with respect to HTLV-1. The "one or a plurality of" for (E6) is, for example, 1 to 105, 1 to 88, 1 to 70, 1 to 52, 1 to 35, 1 to 17, 1 to 14, 1 to 10, 1 to 7, 1 to 3, 1 or 2, or 1 in the amino acid sequence of (E5).

For (E7), the "identity", for example, should be a range in which the protein encoded by the polynucleotide of (E7) exhibits immunogenicity with respect to HTLV-1. The "identity" of (E7) is, for example, at least 70%, at least 75%, at least 80%, at least 85%, at least 90%, at least 95%, at least 96%, at least 97%, at least 98%, or at least 99% relative to the amino acid sequence of (E5).

The nucleic acid that encodes HBZ can be exemplified by polynucleotides selected from the group consisting of the following (F1) to (F6) and (F7):
(F1) a polynucleotide composed of the base sequence with SEQ ID NO: 6 or 8;
(F2) a polynucleotide composed of a base sequence in which one or a plurality of bases have been deleted, inserted, substituted, and/or added in a base sequence of (F1);
(F3) a polynucleotide composed of a base sequence having an at least 80% identity with either base sequence of (F1);
(F4) a polynucleotide composed of a base sequence that is complementary to a polynucleotide that hybridizes under stringent conditions with a polynucleotide composed of either base sequence of (F1);
(F5) a polynucleotide that encodes a protein composed of the amino acid sequence of SEQ ID NO: 5 or 7;
(F6) a polynucleotide that encodes a protein composed of an amino acid sequence in which one or a plurality of amino acids have been deleted, inserted, substituted, and/or added in an amino acid sequence of (F5); and
(F7) a polynucleotide that encodes a protein composed of an amino acid sequence having an at least 80% identity with an amino acid sequence of (F5).

The base sequences with SEQ ID NO: 6 or 8 of (F1) are given below. The base sequences with SEQ ID NO: 6 or 8 are the coding sequences for HBZ composed of the amino acid sequence with SEQ ID NO: 5 or 7. The polynucleotides (F1) with SEQ ID NO: 6 or 8 can be obtained, for example, from HTL V-1.
Polynucleotide (SEQ ID NO: 6) of (F1) 
5'-atggcggcctcagggctgtttcgatgcttgcctgtgtcatgcccggaggacctgctggtggaggaattggtggacgggctattatc

cttggaggaagagttaaaggacaaggaggaggaggaagctgtgcttgacggtttgctatccttagaagaggaaagccgcggccggc

tgcgacggggccctccaggggagaaagcgccacctcgcggggaaacgcatcgtgatcggcagcgacgggctgaggagaagag

gaagcgaaaaaaagagcgggagaaagaggaggaaaagcagaccgctgagtatttgaaaaggaaggaagaggagaaggcacgg

cgcaggaggcgggcggagaagaaggccgctgacgtcgctaggaggaagcaggaagagcaggagcgccgtgagcgcaagtgg

agacaaggggctgagaaggcgaaacagcatagtgctaggaaagaaaaaatgcaggagttggggattgatggctatactagacagtt

ggaaggcgaggtggagtccttggaggctgaacggaggaagttgctgcaggagaaggaggatttgatgggagaggttaattattggc

aggggaggctggaggcgatgtggttgcaataa-3'
Polynucleotide (SEQ ID NO: 8) of (F1)

5'-atggcggcctcagggctgtttcgatgcttgcctgtgtcatgcccggaggacctgctggtggaggaattggtggacgggctattatc

cttggaggaagagttaaaggacaaggaggaggaggaagctgtgcttgacggtttgctatccttagaagaggaaagccgcggccggc

tgcgacggggccctccaggggagaaagcgccacctcgcggggaaacgcatcgtgatcggcagcgacgggccgaggagaagag

gaagcgaaaaaaagagcgggagaaagaggaggaaaagcagattgctgagtatttgaaaaggaaggaagaggagaaggcacggc

gcaggaggcgggcggagaagaaggccgctgacgtcgccaggaggaagcaggaagagcaggagcgccgtgagcgcaagtgga

gacaaggggctgagaaggcgaaacagcatagtgctaggaaagaaaaaatgcaggagttggggattgatggctatactagacagttg

gaaggcgaggtggagtccttggaggctgaacggaggaagttgctgcaggagaaggaggatttgatgggagaggttaattattggca

ggggaggctggaggcgatgtggttgcaataa-3'

For (F2), the "one or a plurality of', for example, should be a range in which the protein encoded by the polynucleotide of (F2) exhibits immunogenicity with respect to HTLV-1. The "one or a plurality of" for (F2) is, for example, 1 to 186, 1 to 156, 1 to 123, 1 to 93, 1 to 60, 1 to 30, 1 to 24, 1 to 18, 1 to 12, 1 to 6, 1 to 3, 1 or 2, or 1 in the base sequences of (F1).

For (F3), the "identity", for example, should be a range in which the protein encoded by the polynucleotide of (F3) exhibits immunogenicity with respect to HTLV-1. The "identity" of (F3) is, for example, at least 70%, at least 75%, at least 80%, at least 85%, at least 90%, at least 95%, at least 96%, at least 97%, at least 98%, or at least 99% relative to the base sequences of (F1).

For (F4), the "polynucleotide that hybridizes", for example, is a polynucleotide that is fully or partially complementary to the polynucleotide of (F1). This hybridization can be detected, for example, by various hybridization assays. There are no particular limitations on the hybridization assay, and, for example, the methods described in "Molecular Cloning: A Laboratory Manual 2nd Ed.", edited by Sambrook et al. [Cold Spring Harbor Laboratory Press (1989)] can also be adopted. The description of the conditions in (A4) above can be adopted for these stringent conditions for (F4).

The polynucleotide of (F5) can be established, for example, by conversion to the corresponding codons based on the amino acid sequence of SEQ ID NO: 5 or 7.

For (F6), the "one or a plurality of", for example, should be a range in which the protein encoded by the polynucleotide of (F6) exhibits immunogenicity with respect to HTLV-1. The "one or a plurality of" for (F6) is, for example, 1 to 62, 1 to 52, 1 to 41, 1 to 31, 1 to 20, 1 to 10, 1 to 8, 1 to 6, 1 to 4, or 1 or 2 in the amino acid sequences of (F5).

For (F7), the "identity", for example, should be a range in which the protein encoded by the polynucleotide of (F7) exhibits immunogenicity with respect to HTLV-1. The "identity" of (F7) is, for example, at least 70%, at least 75%, at least 80%, at least 85%, at least 90%, at least 95%, at least 96%, at least 97%, at least 98%, or at least 99% relative to the amino acid sequences of (F5).

In the present disclosure, the nucleic acid for an immunogenic fragment of Tax or HBZ, for example, should be a range in which the protein encoded by this polynucleotide exhibits immunogenicity with respect to HTLV-1.

The nucleic acid may be composed of deoxynucleotide residues, or may be composed of ribonucleotide residues, or may be composed of both. The nucleic acid may be composed of natural nucleic acid residues, or may be composed of non-natural nucleic acid residues, or may be composed of both. As specific examples, the nucleic acid may contain, for example, DNA, RNA, and/or DNA/RNA composed of natural and/or non-natural nucleic acid residues. The non-natural nucleic acid residues can be exemplified by modified nucleotide residues or modified ribonucleotide residues in which the base, sugar residue, or sugar phosphate backbone of the nucleotide residue has been modified. In the case of modification of the sugar residue, the non-natural nucleic acid residue can be exemplified by cEt (constrained ethyl bicyclic nucleic acid, Ionis Pharmaceuticals, Inc.), LNA (trademark, Locked Nucleic Acid), and ENA (registered trademark, 2'-O,4'-C-Ethylenebridged Nucleic Acid). The nucleic acid may have, for example, a 5' cap at the 5' end.

The nucleic acid may be a single-stranded nucleic acid molecule or a double-stranded nucleic acid molecule.

In the nucleic acid-containing pharmaceutical composition of the present disclosure, the nucleic acid encoding a particular protein as described above, for example, can be designed by substituting the corresponding codons based on the amino acid sequence of the particular protein. The base sequence of the nucleic acid in the pharmaceutical composition of the present disclosure, for example, may be codon optimized.

The various nucleic acids and proteins in the present disclosure, for example, can be synthesized by genetic engineering techniques or organic synthesis techniques, and can also be referred to as synthetic DNA, e.g., cDNA, or synthetic RNA.

In the present disclosure, the nucleic acid preferably is a modified nucleic acid. In a specific example for the case in which the nucleic acid is RNA, a portion or all of the uridine in this RNA is preferably pseudouridine.

In the present disclosure, when the nucleic acid is mRNA, this nucleic acid may contain, e.g., a 5' cap structure, a 5'-UTR, a 3'-UTR, a poly-A sequence, and so forth, at the terminals.

In the present disclosure, the nucleic acid, for example, may form a complex with a lipid complex (lipid nanoparticle).

### <The vector-containing pharmaceutical composition>

In another aspect, the present disclosure relates to a pharmaceutical composition that can induce an immune response to HTLV-1 and particularly to HTLV-1 Gag. The pharmaceutical composition (a vector-containing pharmaceutical composition) of the present disclosure comprises: a vector that contains nucleic acid that encodes human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

The vector-containing pharmaceutical composition of the present disclosure is characterized in that it contains, as an active ingredient, a vector containing nucleic acid that encodes HTLV-1 antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof, with there being no particular limitation on its other features and conditions. The vector-containing pharmaceutical composition of the present disclosure - because it contains, as an active ingredient, a vector containing nucleic acid that encodes HTLV-1 antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof - can induce an immune response to HTLV-1 when used as a pharmaceutical composition. The description of the pharmaceutical composition containing the Gag antigen of HTLV-1 and the description of the nucleic acid-containing pharmaceutical composition can be applied to the vector-containing pharmaceutical composition of the present disclosure, unless specifically indicated otherwise.

With regard to this vector, for example, the nucleic acid that encodes Gag p15 or an immunogenic fragment thereof, Gag p19 or an immunogenic fragment thereof, and/or Gag protein p24 (Gag p24) or an immunogenic fragment thereof is inserted or incorporated in the vector in the vector-containing pharmaceutical composition of the present disclosure. For example, it can also be said that this nucleic acid is functionally linked to the vector. This vector denotes, for example, a nucleic acid molecule that can transport an inserted gene into a target, e.g., a cell and so forth.

The vector, for example, is not particularly limited in its configuration as long as it contains a protein-encoding polynucleotide in such a manner that the protein encoded by the polynucleotide of the nucleic acid can be expressed.

The vector can be constructed, for example, by inserting, into a framework vector (also referred to hereafter as the "basic vector"), a polynucleotide that encodes a protein of the active ingredient in the pharmaceutical composition of the present disclosure, i.e., a nucleic acid of the present disclosure encoding Gag p15 or an immunogenic fragment thereof, Gag p19 or an immunogenic fragment thereof, and/or Gag protein p24 (Gag p24) or an immunogenic fragment thereof. The type of vector is not particularly limited and, for example, can be established as appropriate in conformity with the nature of the recipient target.

The vector, for example, may additionally contain a nucleic acid that encodes the Tax protein of HTLV-1 or an immunogenic fragment thereof and/or the HBZ protein or an immunogenic fragment thereof.

When there are a plurality of proteins for the active ingredient in the pharmaceutical composition of the present disclosure, a single vector may contain a nucleic acid that encodes the plurality of proteins or a plurality of vectors may respectively contain a nucleic acid that encodes one protein or a plurality of proteins.

The vector can be exemplified by viral vectors and non-viral vectors. Examples of viral vectors are baculoviruses; poxviruses such as vaccinia virus, avipox virus, canarypox virus, fowlpox virus, raccoonpox virus, and swinepox virus; adenoviruses such as canine adenovirus; adeno-associated viruses; herpes viruses; and retroviruses. When host transformation is carried out using a heat shock technique for the method of introduction, the vector can be exemplified by binary vectors. Examples of this vector are pETDuet-1, pQE-80L, and pUCP26Km. When carrying out the transformation of bacteria such as Escherichia coli, the vector can be exemplified by pETDuet-1 vector (Novagen), pQE-80L (QIAGEN), pBR322, pB325, pAT153, and pUC8. When carrying out the transformation of yeast, the vector can be exemplified by pYepSec1, pMFa, and pYES2. When carrying out the transformation of insect cells, the vector can be exemplified by pAc and pVL. When carrying out the transformation of mammalian cells, the vector can be exemplified by pCDM8 and pMT2PC.

The vector, for example, preferably has regulatory sequences that regulate the expression of the polynucleotide encoding the Gag p15 or immunogenic fragment thereof, Gag p19 or immunogenic fragment thereof, and/or Gag p24 or immunogenic fragment thereof of the present disclosure, and that regulate the expression of the Gag p15 or immunogenic fragment thereof, Gag p19 or immunogenic fragment thereof, and/or Gag p24 or immunogenic fragment thereof of the present disclosure, encoded by the polynucleotide for the Gag p15 or immunogenic fragment thereof, Gag p19 or immunogenic fragment thereof, and/or Gag p24 or immunogenic fragment thereof of the present disclosure. The regulatory sequence can be exemplified by promoters, terminators, enhancers, polyadenylation signal sequences, and replication origins (ori). The disposition of the regulatory sequences in the vector is not particularly limited. The regulatory sequences can be disposed in the vector based on known methods, insofar as the disposition enables functional regulation of the expression of the polynucleotide encoding the Gag p15 or immunogenic fragment thereof, Gag p19 or immunogenic fragment thereof, and/or Gag p24 or immunogenic fragment thereof of the present disclosure, and the expression of the thereby encoded Gag p15 or immunogenic fragment thereof, Gag p19 or immunogenic fragment thereof, and/or Gag p24 or immunogenic fragment thereof of the present disclosure. For example, a sequence already included in the vector may be utilized as a regulatory sequence, or an additional regulatory sequence may be inserted into the vector, or a regulatory sequence present in the vector may be replaced with another regulatory sequence.

This vector, for example, may be used to produce the protein in the pharmaceutical composition of the present disclosure. **In** this case, protein production can be carried out by introducing the vector into a host and causing the expression of the protein encoded by the vector.

The type of vector is not particularly limited and, for example, can be determined as appropriate in conformity with the nature of the host. Specifically, when the vector is to be synthesized by genetic engineering techniques, vector synthesis proceeds first, for example, by designing and synthesizing the nucleic acid that encodes the Gag p15 or immunogenic fragment thereof, Gag p19 or immunogenic fragment thereof, and/or Gag p24 or immunogenic fragment thereof of the present disclosure. The design and synthesis, for example, can be performed by PCR using as the template, for example, a vector that contains a nucleic acid encoding the Gag p15 or immunogenic fragment thereof, Gag p19 or immunogenic fragment thereof, and/or Gag p24 or immunogenic fragment thereof of the present disclosure, and using primers designed to enable synthesis of the desired region of the nucleic acid. The resulting nucleic acid is then ligated into an appropriate vector to obtain a recombinant vector for protein expression, and a transformant can be obtained by introducing this recombinant vector into a host so as to enable expression of a target gene (Sambrook J. et al., Molecular Cloning, A Laboratory Manual (4th edition) (Cold Spring Harbor Laboratory Press (2012))).

The host used for transformation should be capable of expressing the gene of interest, but is not otherwise particularly limited, and examples thereof include non-human hosts such as microorganisms, animal cells, insect cells, and cultured cells of the preceding; isolated human cells and cultured cells thereof; and mammalian cells. Examples of the prokaryotic organisms are bacteria such as bacteria in genus Escherichia such as Escherichia coli and bacteria in genus Pseudomonas such as Pseudomonas putida. The eukaryotic organisms can be exemplified by yeasts such as Saccharomyces cerevisiae. The animal cells can be exemplified by COS cells and CHO cells, while the insect cells can be exemplified by Sf9 and Sf21.

The method of introducing the vector into a host is not particularly limited and known methods can be used. The introduction method can be established as appropriate in conformity with, for example, the nature of the host. The introduction method can be exemplified by introduction using a gene gun such as a particle gun, the calcium phosphate method, the polyethylene glycol method, lipofection using liposomes, electroporation, ultrasonic nucleic acid transfer, the DEAE-dextran method, direct injection using, e.g., a glass microtube, the hydrodynamic method, the cationic liposome method, methods using transfection adjuvants, Agrobacterium-mediated methods, and protoplast methods. The liposomes can be exemplified by lipofectamine and cationic liposomes, and the transfection adjuvants can be exemplified by atelocollagen, nanoparticles, and polymers. When the host is a microorganism, for example, a method mediated by, e.g., E. coli or Ps. putida, is preferred among the preceding. The polynucleotide for the Gag p15 or immunogenic fragment thereof, Gag p19 or immunogenic fragment thereof, and/or Gag p24 or immunogenic fragment thereof of the present disclosure may be introduced into a host, for example, using the vector of the present disclosure.

In a production method of the present disclosure, for example, a transformant as described in the preceding is cultured and the Gag p15 or immunogenic fragment thereof, Gag p19 or immunogenic fragment thereof, and/or Gag p24 or immunogenic fragment thereof of the present disclosure is collected from the resulting culture material. The culture material may be the culture supernatant or may be the transformant, e.g., cultured cells or cultured bacterial cells, or a processed material or disrupted material thereof.

After culturing, when the Gag p15 or an immunogenic fragment thereof, Gag p19 or an immunogenic fragment thereof, and/or Gag p24 or an immunogenic fragment thereof of the present disclosure has been produced within the host, in a production method of the present disclosure, for example, the Gag p15 or an immunogenic fragment thereof, Gag p19 or an immunogenic fragment thereof, and/or Gag p24 or an immunogenic fragment thereof is extracted by disruption of the host. When the Gag p15 or an immunogenic fragment thereof, Gag p19 or an immunogenic fragment thereof, and/or Gag p24 or an immunogenic fragment thereof of the present disclosure has been produced or secreted outside the host, in a production method of the present disclosure, for example, the culture fluid is used as such or the host is removed by, e.g., centrifugation. The protein, e.g., Gag p15 or an immunogenic fragment thereof, Gag p19 or an immunogenic fragment thereof, and/or Gag p24 or an immunogenic fragment thereof, can then be isolated or purified in a production method of the present disclosure, for example, by using the general biochemical methods used to isolate and purify proteins, and specifically using a single one of, or a suitable combination of, concentration using an ultrafiltration membrane; salting out, e.g., ammonium sulfate precipitation; chromatography using various columns, e.g., gel filtration, ion-exchange chromatography, affinity chromatography, and so forth.

### <Method of prevention>

In another aspect, the present disclosure provides the treatment and/or prevention of HTLV-1-associated diseases, or a method for preventing the onset of ATL, the aggravation of ATL, or the recurrence of ATL. This treatment and/or prevention of HTLV-1-associated diseases of the present disclosure, or method of the present disclosure for preventing the onset of ATL, the aggravation of ATL, or the recurrence of ATL, is characterized by the use in a subject of the HTLV-1 Gag antigen-containing pharmaceutical composition of the present disclosure, the nucleic acid-containing pharmaceutical composition of the present disclosure, and/or the vector-containing pharmaceutical composition of the present disclosure, while the other features and conditions are not particularly limited. The prevention method of the present disclosure - due to the presence as an active ingredient of the Gag p15 or an immunogenic fragment thereof, Gag p19 or an immunogenic fragment thereof, and/or Gag p24 or an immunogenic fragment thereof, or a nucleic acid encoding same - can induce an immune response to HTLV-1 when a subject is treated. For example, the descriptions of the HTLV-1 Gag antigen-containing pharmaceutical composition of the present disclosure, the nucleic acid-containing pharmaceutical composition of the present disclosure, and the vector-containing pharmaceutical composition of the present disclosure can be cited for the prevention method of the present disclosure.

Since the prophylactic method of the present disclosure, for example, can induce an immune response against HTL V-1, it can also be referred to as, for example, a vaccination method against HTL V-1, a method for inducing protective immunity, a method for stimulating an immune response, or a method for inducing an immune response. In addition, the prevention method of the present disclosure, for example, can also be referred to as the prevention and/or treatment of HTLV-1-associated diseases.

In the prophylactic method of the present disclosure, immunity to HTLV-1 can be conferred on a subject by administering the above-described active ingredient to the subject and inducing an immune response in the subject. As a consequence, the prevention method of the present disclosure, for example, includes a step (administration step) of administering, to a subject, the HTLV-1 Gag antigen-containing pharmaceutical composition of the present disclosure, the nucleic acid-containing pharmaceutical composition of the present disclosure, and/or the vector-containing pharmaceutical composition of the present disclosure, containing the active ingredient as described in the preceding. As a consequence, in the prevention method of the present disclosure, for example, an immune response is induced in the subject against Gag p15 or an immunogenic fragment thereof, Gag p19 or an immunogenic fragment thereof, and/or Gag p24 or an immunogenic fragment thereof and a cellular immune response, by T cells and so forth, is produced against Gag p15 or an immunogenic fragment thereof, Gag p19 or an immunogenic fragment thereof, and/or Gag p24 or an immunogenic fragment thereof.

This administration step is carried out one time or a plurality of times in the prevention method of the present disclosure. The description of the pharmaceutical composition of the present disclosure can be cited for the administration conditions in this administration step.

### <Use>

In another aspect, the present disclosure relates to use of the HTLV-1 Gag antigen-containing pharmaceutical composition of the present disclosure, the nucleic acid-containing pharmaceutical composition of the present disclosure, and/or the vector-containing pharmaceutical composition of the present disclosure for use in a method for preventing the onset of ATL, the aggravation of ATL, or the recurrence of ATL. The present disclosure relates to use of the HTLV-1 Gag antigen-containing pharmaceutical composition of the present disclosure, the nucleic acid-containing pharmaceutical composition of the present disclosure, and/or the vector-containing pharmaceutical composition of the present disclosure for use in the production of a vaccine for use in the suppression of the onset of ATL, the aggravation of ATL, or the recurrence of ATL. For example, the descriptions of the HTLV-1 Gag antigen-containing pharmaceutical composition of the present disclosure, the nucleic acid-containing pharmaceutical composition of the present disclosure, the vector-containing pharmaceutical composition of the present disclosure, and the prevention method of the present disclosure can be cited for the use of the HTLV-1 Gag antigen-containing pharmaceutical composition of the present disclosure, the nucleic acid-containing pharmaceutical composition of the present disclosure, and/or the vector-containing pharmaceutical composition of the present disclosure.

### Examples

The present disclosure is described in detail in the following using examples, but the present disclosure is not limited to or by the embodiments described in the examples. Unless specifically indicated otherwise, the commercial reagents, kits, and so forth were used in accordance with their protocols.

### [Example 1]

It was confirmed that Gag p15, Gag p19 and/or Gag p24, which are active ingredients in the pharmaceutical composition of the present disclosure, can induce an immune response against HTLV-1.

### (1) Investigation of HTLV-1 antigens

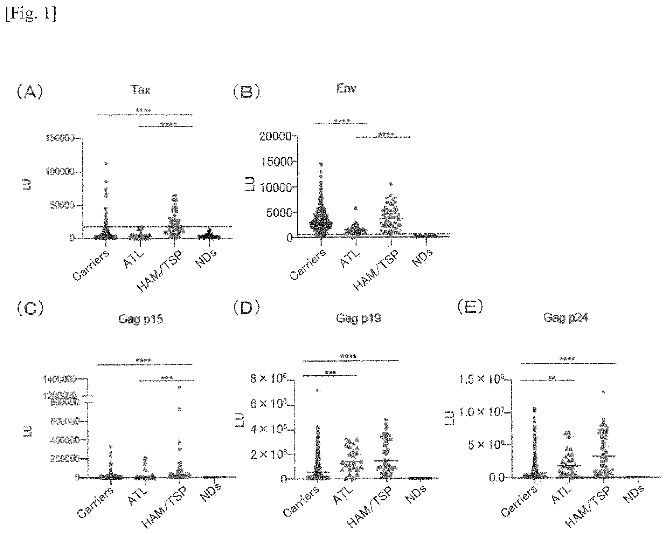

Serum or plasma from HTLV-1-infected individuals, ATL patients, and so forth was used to study antibody responses with HTLV-1 antigens. Specifically, a luciferase immunoprecipitation procedure (LIPS assay) was carried out. Serum or plasma was obtained from HTLV-1-infected individuals, ATL patients, HAM/TSP patients, and HTLV-1-infection-free individuals. All clinical samples were collected after informed consent was obtained in writing in accordance with the Helsinki Declaration. The experiments described in the example were approved by the Institutional Ethics Committee of Kumamoto University (accession numbers: G489, G499, and E2214). The antigens used were Tax (SEQ ID NO: 17), Env (SEQ ID NO: 18), Gag p15 (SEQ ID NO: 19), Gag p19 (SEQ ID NO: 20), and Gag p24 (SEQ ID NO: 21). Vectors expressing a fusion protein between the particular protein and NanoLuc were fabricated; after this fabrication, the vectors were introduced into a 293T cell line; and the cell lysate of these cells was used as the antigen. A preparation was carried out by mixing antigen corresponding to 10⁷ counts, LIPS buffer, and 1 µL of the serum or plasma on a 96-well plate to provide a total of 100 µL. After this preparation, the plate was incubated using a plate mixer and using conditions of room temperature (hereinafter, approximately 24°C) for 30 minutes. After this incubation, Protein A/G magnetic beads (Thermo Fisher Scientific) were washed 3 to 5 times with LIPS buffer using a magnetic plate. After washing, the magnetic beads were resuspended in LIPS buffer. After resuspension, 10 µL of the magnetic beads was added to each well of the plate. After this addition, the plate was incubated using a plate mixer under conditions of room temperature for 30 minutes. After the incubation, in order to concentrate the immune complexes consisting of the antibodies in the patient sample and the NanoLuc-viral antigen fusion protein, the plate was placed on a magnetic plate and the pellet in each well was washed five times with 150-200 µL of LIPS buffer. After the washing, the LIPS buffer used for washing was removed, and 100 µL of Nano-Glo luciferase assay substrate (Promega) was added to each well. After the addition, the luminescence was measured using a luminometer (Promega). The results are reported in Fig. 1.
Amino acid sequence (SEQ ID NO: 17) of Tax 
MAHFPGFGQSLLFGYPVYVFGDCVQGDWCPISGGLCSARLHRHALLATCPEHQITW
DPIDGRVIGSALQFLIPRLPSFPTQRTSKTLKVLTPPITHTTPNIPPSFLQAMRKYSPFRN
GYMEPTLGQHLPTLSFPDPGLRPQNLYTLWGGSVVCMYLYQLSPPITWPLLPHVIFC
HPGQLGAFLTNVPYKRIEELLYKISLTTGALIIILPEDCLPTTLFQPARAPVTLTAWQNG
LLPFHSTLTTPGLIWTFTDGTPMISGPCPKDGQPSLVLQSSSFIFHKFQTKAYHPSFLLS
HGLIQYSSFHSLHLLFEEYTNIPISLLFNEKEADDNDHEPQISPGGLEPPSEKHFRETEV
Amino acid sequence (SEQ ID NO: 18) of Env 
MGKFLATLILFFQFCPLIFGDYSPSCCTLTIGVSSYHSKPCNPAQPVCSWTLDLLALSA
DQALQPPCPNLVSYSSYHATYSLYLFPHWIKKPNRNGGGYYSASYSDPCSLKCPYLG
CQSWTCPYTGAVSSPYWKFQHDVNFTQEVSRLNINLHFSKCGFPFSLLVDAPGYDPI
WFLNTEPSQLPPTAPPLLPHSNLDHILEPSIPWKSKLLTLVQLTLQSTNYTCIVCIDRAS
LSTWHVLYSPNVSVPSSSSTPLLYPSLALPAPHLTLPFNWTHCFDPQIQAIVSSPCHNS
LILPPFSLSPVPTLGSRSRRAVPVAVWLVSALAMGAGVAGGITGSMSLASGKSLLHE
VDKDISQLTQAIVKNHKNLLKIAQYAAQNRRGLDLLFWEQGGLCKALQEQCCFLNI
TNSHVSILQERPPLENRVLTGWGLNWDLGLSQWAREALQTGITLVALLLLVILAGPCI
LRQLRHLPSRVRYPHYSLINPESSL
Amino acid sequence of Gag p15 (SEQ ID NO: 19) 
VVQPKKPPPNQPCFRCGKAGHWSRDCTQPRPPPGPCPLCQDPTHWKRDCPRLKPTIP

EPEPEEDALLLDLPADIPHPKNLHRGGGLTSPPTLQQVLPNQDPASIL
Amino acid sequence of Gag p19 (SEQ ID NO: 20) 
MGQIFSRSASPIPRPPRGLAAHHWLNFLQAAYRLEPGPSSYDFHQLKKFLKIALETPV
WICPINYSLLASLLPKGYPGRVNEILHILIQTQAQIPSRPAPPPSSPTHDPPDSDPQIPPP
YVEPTAPQVL
Amino acid sequence of Gag p24 (SEQ ID NO: 21)

PVMHPHGAPPNHRPWQMKDLQAIKQEVSQAAPGSPQFMQTIRLAVQQFDPTAKDL
QDLLQYLCSSLVASLHHQQLDSLISEAETRGITGYNPLAGPLRVQANNPQQQGLRRE
YQQLWLAAFAALPGSAKDPSWASILQGLEEPYHAFVERLNIALDNGLPEGTPKDPIL
RSLAYSNANKECQKLLQARGHTNSPLGDMLRACQTWTPKDKTKVL

Fig. 1 contains graphs that report the results of the LIPS assays. In Fig. 1, the horizontal axis indicates the type of subject and the vertical axis indicates the luminescence intensity. Fig. 1(A) shows the antibody response to Tax, Fig. 1(B) shows the antibody response to Env, Fig. 1(C) shows the antibody response to Gag p15, Fig. 1(D) shows the antibody response to Gag p19, and Fig. 1(E) shows the antibody response to Gag p24. As shown in Fig. 1, it was found that the antibody responses to Tax and Env were lower in ATL patients than in HTLV-1-infected individuals. It was also found that the antibody responses to Gag p19 and Gag p24 were higher in ATL patients than in HTLV-1-infected individuals.

### (2) Investigation of Gag expression

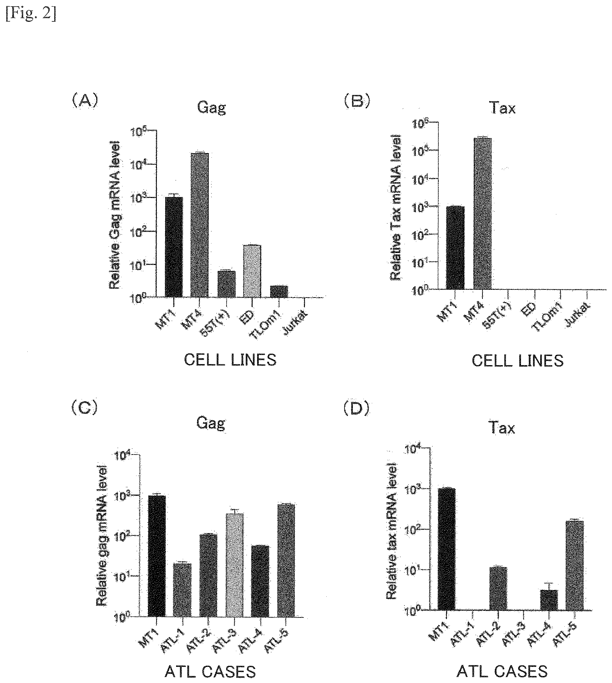

In Example 1(1), the ATL patients had low antibody responses to Tax and high antibody responses to Gag, and the gene expression of Tax and Gag was examined as a consequence. Specifically, quantitative reverse transcription PCR (RT-qPCR) was carried out. For the HTLV-1 cases, MT1 cells, MT4 cells, 55T(+) cells, ED cells, and TLOm1 cells, which are ATL cell lines, and Jurkat cells, an HTLV-1-negative T cell line, were used. For the ATL cases, MT1 cells and sera collected from ATL patients (ATL-1, ATL-2, ATL-3, ATL-4, and ATL-5) were used. The total RNA was extracted from each of these cells using TRIzol Reagent (Invitrogen). After extraction, cDNA was synthesized using SuperScript IV Reverse Transcriptase (Invitrogen). Gag cDNA was prepared using the Gag cDNA synthesis primers indicated below. Tax cDNA and 18SrRNA were synthesized using random primers. After the synthesis, Gag expression was measured using Taqman real-time PCR. Tax expression was measured using SYBR-green real-time PCR. The 18SrRNA was quantified using Taqman Gene Expression Assays (Hs99999901_sl, Applied Biosystems). The amplification conditions were conditions for 1 cycle = 50°C for 2 minutes, 95°C for 10 minutes, 95°C for 15 seconds, and 60°C for 1 minute, and a total of 40 cycles were carried out. These results are shown in Fig. 2.
· cDNA synthesis primer for Gag
primer 1 (SEQ ID NO: 22)
   5'- TGCAGGATATGGGCC-3'.
· primer set for Gag
   primer 2 (SEQ ID NO: 23)
      5'-AGTACCTTTGCTCCTCCCTC-3'
   primer 3 (SEQ ID NO: 24)
      5'-TAATACCTCGGGTTTCGGCC-3'
· probe for Gag
   probe (SEQ ID NO: 25)
   5'-TTCCCTCCATCACCAGCTAGATAGCCT-3'
· primer set for Tax
   primer 4 (SEQ ID NO: 26)
      5'-CCGCCGATCCCAAAGAA-3'
   primer 5 (SEQ ID NO: 27)
      5'-CCTGTCCAAACCCTGGGAA-3'

Fig. 2 contains graphs showing the results of RT-qPCR. In Fig. 2, the horizontal axis indicates the type of sample and the vertical axis indicates the relative mRNA expression level. Fig. 2(A) reports the expression level of Gag mRNA in each individual cell line, Fig. 2(B) reports the expression level of Tax mRNA in each individual cell line, Fig. 2(C) reports the expression level of Gag mRNA in the ATL case samples, and Fig. 2(D) reports the expression level of Tax mRNA in the ATL case samples. As shown in Fig. 2, for ATL cells and ATL case samples, it was shown that Gag is expressed even in ATL cells or ATL case samples in which Tax is not being expressed. These results demonstrated the presence of a Tax-independent Gag expression mechanism.

### (3) Investigation of Gag expression in Tax-nonexpressing cells

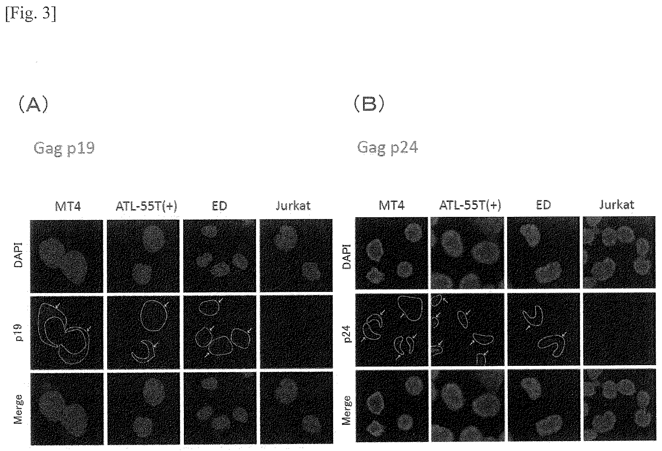

Whether Gag was expressed in cells that did not express Tax was then investigated. Specifically, a proximity ligation assay (PLA) was performed. PLA was performed using a Duolink PLA kit (Sigma-Aldrich) according to the manufacturer's instructions. The cells used were MT4 cells, ATL-55T(+) cells, and ED cells, which are ATL cell lines, while Jurkat cells, a T cell line, were used as a negative control. The cells were concentrated onto glass coverslips using a Cytospin (Cytospin 2, Shandon). After concentration, fixing was carried out by the addition of 4% paraformaldehyde using conditions of 15 minutes at room temperature. After fixation, a permeabilization treatment was carried out by the addition of 0.2% Triton (registered trademark) X-100 using conditions of 15 minutes at room temperature. After the permeabilization treatment, blocking was carried out using Duolink In Situ PLA Probe-Blocking Solution. After blocking, the cells were stained with antibodies. The following were used for staining: mouse anti-HTLV-1 p24 monoclonal antibody (clone 46/3.24.4, ZeptoMetrix), rabbit polyclonal antibody against HTLV-1 p24 Gag (Cat No. 5418, ABL), or mouse anti-HTLV-1 p19 Gag monoclonal antibody (Cat No. 0801003, ZeptoMetrix). After staining, the glass coverslip was sealed with DAPI-containing In Situ Mounting Medium (Sigma-Aldrich). After sealing, the cells were observed using a Nikon C2 confocal microscope (Nikon). The results are reported in Fig. 3.

Photographs showing the results of the proximity ligation assay are given in Fig. 3. Fig. 3(A) shows the results for the Gag p19 antibody. Fig. 3(B) shows the results for the Gag p24 antibody. In Fig. 3, the upper row shows the results of DAPI staining, the middle row shows the results of Gag p19 or Gag p24 staining, and the lower row shows the results of DAPI and Gag p19 or Gag p24 staining (Merge). In Fig. 3, the area surrounded by the dashed line indicated by the arrows shows the expression of Gag p19 or Gag p24. As shown in Fig. 3(A), it was found that Gag p19 was expressed not only in Tax-expressing MT4 cells, but also in ATL-55T(+) cells and ED cells, which do not express Tax. Furthermore, as shown in Fig. 3(B), Gag p24 was found to be expressed not only in Tax-expressing MT4 cells, but also in ATL-55T(+) cells and ED cells, which do not express Tax. These results demonstrated that Gag p19 and p24 are expressed in ATL cells, regardless of the presence/absence of Tax expression.

### (4) Investigation of the cellular immune response due to Gag in ATL patients after hematopoietic stem cell transplantation

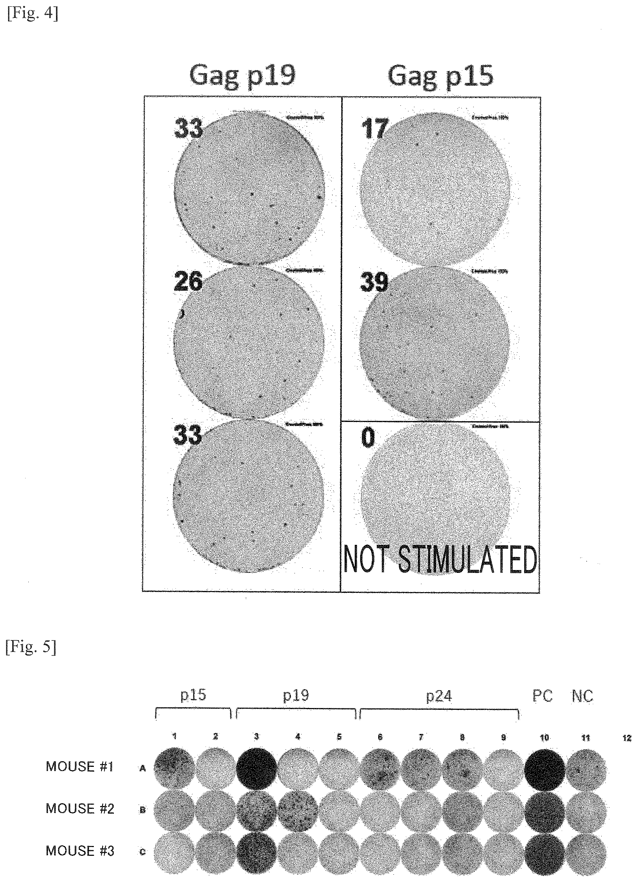

An investigation was then carried out into whether a cellular immune response is induced by Gag in ATL patients who had undergone hematopoietic stem cell transplantation and had maintained long-term remission. Specifically, an ELISpot assay was used. Overlapping peptides with a length of 9 amino acids (offset: 1 amino acid) were designed based on the amino acid sequence of Gag (GenBank accession number AAA85841.1, SEQ ID NO: 1). The Gag p19 peptide was designed starting from the second amino acid of SEQ ID NO: 1. 97 peptides for Gag p15 were pooled as p15-1 (p15₁₋₅₀) and p15-2 (p15₅₁₋₉₇). 121 peptides for Gag p19 were pooled as p19-1 (p19₁₋₅₀), p19-2 (p19₅₁₋₁₀₀), and p19-3 (p19₁₀₁₋₁₂₁). Peripheral blood mononuclear cells (PBMCs) were collected from ATL patients who had undergone hematopoietic stem cell transplantation and had maintained long-term remission. These were submitted to an ELISpot assay using a human IFN-γ ELISpot kit (MABTECH). The PBMCs were seeded onto an ELISpot plate. After seeding, 0.25 µmol/L pooled peptide and 1 µg/mL Purified NA/LE Mouse Anti-Human CD28 antibody (Cat No: 555725, BD Biosciences) were added. Stimulation was carried out for 6 hours after the addition. Spots for IFN-γ-producing cells were developed using an AP Conjugate Kit (Bio-Rad) and were counted using an ImmunoSpot S6 Analyzer (Cellular Technology Limited). These results are given in Fig. 4.

Photographs showing the results of the ELISpot assays are given in Fig. 4. In Fig. 4, the left shows the case of stimulation with Gag p19 peptide. In Fig. 4, the upper and middle panels on the right show the case of stimulation with Gag p15 peptide, and the lower panel on the right panel shows the case of no stimulation. As shown in Fig. 4, spots were formed when stimulation with Gag p19 or Gag p15 peptide was carried out. These results demonstrated that IFN-γ was produced due to Gag p19 or Gag p15 in PBMCs from ATL patients who had undergone hematopoietic stem cell transplantation and had maintained long-term remission. In addition, the induction of a cellular immune response was suggested by the production of IFN-γ.

### (5) Investigation of the cellular immune response due to Gag in mice

An investigation was then carried out into whether a cellular immune response was induced in mice immunized with recombinant Gag protein. Specifically, an ELISpot assay was used. Gag p15 peptides and Gag p19 peptides were designed and pooled using the same method as in Example 1(4). Gag p24 peptides were designed using the same method as in Example 1(4). 206 Gag p24 peptides were pooled as p24-1 (p24₁₋₅₁), p24-2 (p24₅₂₋₁₀₃), p24-3 (p24₁₀₄₋₁₅₄), and p24-4 (p24₁₅₅₋₂₀₆). Mice (C57BL/6J, The Jackson Laboratory Japan, Inc.) were immunized with the Gag protein, and after 14 days splenocytes were collected from the mice. The ELISpot procedure was carried out using the same method as in Example 1(4), except that the splenocytes collected from the mice were used as samples.

Photographs showing the results of the ELISpot assays are given in Fig. 5. In Fig. 5, the top row (A) shows the results for splenocytes from mouse 1, the middle row (B) shows the results for splenocytes from mouse 2, and the bottom row (C) shows the results for splenocytes from mouse 3. In Fig. 5 from left to right, 2 columns (Gag p15-1 and 2, respectively, p15-1 (p15₁₋₅₀ and p15-2 (p15₅₁₋₉₇)) show the case of stimulation with Gag p15; 3 columns (Gag p19-3 to 5, respectively, p19-1 (p19₁₋₅₀), p19-2 (p19₅₁₋₁₀₀), and p19-3 (p19₁₀₁₋₁₂₁)) show the case of stimulation with Gag p19; 4 columns (Gag p24-6 to 9, respectively, p24-1 (p24₁₋₅₁), p24-2 (p24₅₂₋₁₀₃), p24-3 (p24₁₀₄₋₁₅₄), and p24-4 (p24₁₅₅₋₂₀₆)) show the case of stimulation with Gag p24; 1 column (10) shows the positive control; and 1 column (11) shows the negative control. As shown in Fig. 5, spots were formed when stimulation with Gag p15, Gag p19, or Gag p24 peptides was carried out. In particular, spot formation was substantial for stimulation with the Gag p19-1 (p19₁₋₅₀) peptides. These results demonstrated that IFN-γ was produced due to Gag p15, Gag p19, or Gag p24 in mice immunized with Gag proteins. The induction of a cellular immune response was suggested by this production of IFN-γ.

### [Example 2]

### (1) Construction of template DNA

In order to construct the template plasmid DNA used for in vitro transcription (IVT), a plasmid was synthesized having a DNA fragment in which the following were linked in sequence: T7 promoter sequence (TAATACGACTCACTATA, SEQ ID NO: 28), 5'-UTR sequence (AGGAAATAAGAGAGAAAAGAAGAGTAAGAAGAAATATAAGA, SEQ ID NO: 29), Kozak sequence (GCCACC, SEQ ID NO: 30), the particular antigen sequence (Gag antigen), and 3'-UTR sequence 
(TAAGCTGCCTTCTGCGGGGCTTGCCTTCTGGCCATGCCCTTCTTCTCTCCCTTGCA

CCTGTACCTCTTGGTCTTTGAATAAAGCCTGAGTAGGAAGGCGG, SEQ ID NO: 31).
 The following were added to nuclease-free water (210 µL) in which 1 µg of the plasmid was dissolved: Q5 Hot Start High-Fidelity 2X Master Mix (250 µL, NEB# M0494L), 10 µM sense primer (20 µL), and 10 µM poly-T-containing antisense primer (20 µL). After incubation at 95°C for 1 minute, PCR amplification of the template DNA was carried out by executing 35 cycles of 95°C for 30 seconds, 60°C for 30 seconds, and 72°C for 3 minutes and an additional incubation for 5 minutes at 72°C. After the reaction, purification was performed using a NucleoSpin Gel and PCR Clean Up Kit (TaKaRa #U0609A) to obtain template DNA having the desired antigen sequence (SEQ ID NO: 32).
<SEQ ID NO: 32> Template DNA containing the sequence for Gag antigen

TAATACGACTCACTATAAGGAAATAAGAGAGAAAAGAAGAGTAAGAAGAAATA

TAAGAGCCACCATGGGCCAGATCTTCTCTCGCTCTGCTTCTCCTATTCCTCGCCCT

CCTCGCGGACTTGCTGCTCACCACTGGCTTAACTTCCTGCAGGCTGCTTACCGCCT

GGAACCTGGCCCTAGCAGCTACGACTTCCACCAGCTGAAGAAGTTCCTGAAGAT

CGCCCTGGAGACCCCCGTGTGGATCTGCCCCATCAACTACAGCCTGCTGGCTAGC

CTGCTGCCTAAAGGCTACCCTGGCCGCGTGAACGAGATCCTGCACATCCTGATCC

AGACCCAGGCCCAGATCCCTTCTCGCCCTGCTCCTCCTCCTCCTTCTTCTCCTACC

CACGATCCCCCCGATAGCGATCCCCAGATCCCCCCTCCTTACGTGGAACCTACCG

CTCCTCAGGTGCTGCCTGTGATGCACCCTCACGGCGCTCCTCCTAATCACCGCCC

TTGGCAGATGAAGGACCTGCAGGCCATCAAACAGGAAGTGAGCCAGGCTGCTCC

CGGCAGCCCCCAGTTTATGCAGACCATCCGCCTGGCTGTGCAGCAGTTCGATCCC

ACCGCTAAAGATCTGCAGGACCTGCTGCAGTACCTGTGCAGCAGCCTGGTGGCT

AGCCTGCACCACCAGCAGCTGGATAGCCTGATCAGCGAAGCCGAAACCCGCGGC
ATCACCGGCTACAATCCTCTGGCTGGCCCTCTGCGCGTGCAGGCTAACAACCCTC
AGCAGCAGGGACTGCGCCGCGAATACCAGCAGCTGTGGCTGGCTGCTTTTGCTG
CTCTGCCCGGCAGCGCTAAAGATCCCAGCTGGGCTAGCATCCTGCAGGGCCTGG
AAGAACCCTACCACGCCTTCGTGGAGCGCCTGAACATCGCCCTGGACAACGGCC
TGCCTGAAGGCACCCCTAAAGATCCTATCCTGCGCAGCCTGGCCTACAGCAACGC
CAACAAGGAGTGCCAGAAGCTGCTGCAGGCTCGCGGACACACCAATAGCCCTCT
GGGAGATATGCTGCGCGCTTGCCAGACCTGGACCCCCAAGGACAAGACCAAGGT
GCTGGTGGTGCAGCCCAAGAAGCCTCCTCCTAACCAGCCTTGCTTTCGCTGCGGC
AAAGCTGGACACTGGAGCCGCGATTGCACACAGCCTCGCCCTCCTCCTGGACCTT
GCCCTCTTTGCCAGGATCCTACCCACTGGAAGCGCGACTGCCCTCGCCTGAAACC
TACCATCCCTGAACCTGAACCTGAAGAAGACGCTCTGCTGCTGGATCTGCCCGCT
GATATCCCCCACCCCAAGAACAGCATCGGCGGCGAGGTGTGATAAGCTGCCTTC
TGCGGGGCTTGCCTTCTGGCCATGCCCTTCTTCTCTCCCTTGCACCTGTACCTCTT
GGTCTTTGAATAAAGCCTGAGTAGGAAGGCGG

### (2) Preparation of mRNA

mRNA was prepared by in vitro transcription (IVT) using each of the obtained template DNAs. 300 µg/mL template DNA (80 µL), 100 mM CleanCap AG (32 µL, TriLink catalog #N-7113), 100 mM ATP (40 µL, TriLink catalog #N-1510), 100 mM CTP (40 µL, TriLink catalog #N-1511), 100 mM GTP (40 µL, TriLink catalog #N-1512), 100 mM N1-methyl-ψ-Uridine-5'-triphosphate (40 µL), UltraPure DNase/RNase-Free Distilled Water (405.6 µL, Thermo Fisher catalog #10977015), T7 Transcription 10x buffer (80 µL), RNase inhibitor (20 µL, NEB, catalog #M0314L), Yeast Inorganic pyrophosphatase (16 µL, NEB, catalog #M2403L), and T7 RNA Polymerase (6.4 µL, Roche catalog #08140669103) were mixed and incubated at 37°C for 3 hours. RNase-Free DNase I (24 µL, TaKaRa catalog #2270A) was mixed in and incubation was carried out for 30 minutes at 37°C. 10x phosphatase buffer (96 µL, NEB, catalog #B0289S) and Antarctic phosphatase (48 µL, NEB, catalog #M0289L) were additionally mixed in and incubation was carried out for 30 minutes at 37°C. 8 M LiCl solution (484 µL, Sigma-Aldrich catalog catalog #L7026) was mixed in and, after standing for at least one hour at -20°C, centrifugation was carried out (4°C, 15,000 rpm, 30 minutes) and the supernatant was discarded. The following operation was then carried out three times: addition of 75% ethanol, centrifugation (4°C, 15,000 rpm, 5 minutes), and discard of the supernatant. The resulting precipitate was dissolved in nuclease-free water and purified using an RNeasy Maxi kit (Qiagen catalog #75162) in accordance with the accompanying manual. Alternatively, the resulting precipitate was treated with RNase III (NEB, catalog #M0245S) for 10 minutes at room temperature and was then purified with an Oligo dT column (Sartorius, catalog #311.1218-2). As a result, mRNA (SEQ ID NO: 33) expressing protein having the amino acid sequence (SEQ ID NO: 1) was obtained.

A cap structure and polyA sequence were added to each mRNA. The cap structure is the structure given below for CleanCap (Cap1) (Fujifilm Wako Pure Chemical Industries, Ltd.). 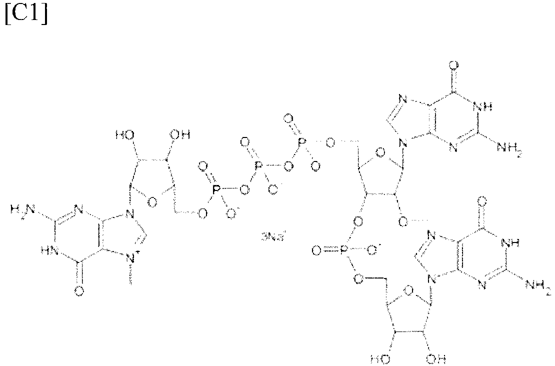
<SEQ ID NO: 33> Gag mRNA 
AGGAAAUAAGAGAGAAAAGAAGAGUAAGAAGAAAUAUAAGAGCCACCAUGGG
CCAGAUCUUCUCUCGCUCUGCUUCUCCUAUUCCUCGCCCUCCUCGCGGACUUG
CUGCUCACCACUGGCUUAACUUCCUGCAGGCUGCUUACCGCCUGGAACCUGGC
CCUAGCAGCUACGACUUCCACCAGCUGAAGAAGUUCCUGAAGAUCGCCCUGGA
GACCCCCGUGUGGAUCUGCCCCAUCAACUACAGCCUGCUGGCUAGCCUGCUGC
CUAAAGGCUACCCUGGCCGCGUGAACGAGAUCCUGCACAUCCUGAUCCAGACC
CAGGCCCAGAUCCCUUCUCGCCCUGCUCCUCCUCCUCCUUCUUCUCCUACCCAC
GAUCCCCCCGAUAGCGAUCCCCAGAUCCCCCCUCCUUACGUGGAACCUACCGC
UCCUCAGGUGCUGCCUGUGAUGCACCCUCACGGCGCUCCUCCUAAUCACCGCC
CUUGGCAGAUGAAGGACCUGCAGGCCAUCAAACAGGAAGUGAGCCAGGCUGCU
CCCGGCAGCCCCCAGUUUAUGCAGACCAUCCGCCUGGCUGUGCAGCAGUUCGA
UCCCACCGCUAAAGAUCUGCAGGACCUGCUGCAGUACCUGUGCAGCAGCCUGG
UGGCUAGCCUGCACCACCAGCAGCUGGAUAGCCUGAUCAGCGAAGCCGAAACC
CGCGGCAUCACCGGCUACAAUCCUCUGGCUGGCCCUCUGCGCGUGCAGGCUAA
CAACCCUCAGCAGCAGGGACUGCGCCGCGAAUACCAGCAGCUGUGGCUGGCUG
CUUUUGCUGCUCUGCCCGGCAGCGCUAAAGAUCCCAGCUGGGCUAGCAUCCUG
CAGGGCCUGGAAGAACCCUACCACGCCUUCGUGGAGCGCCUGAACAUCGCCCU
GGACAACGGCCUGCCUGAAGGCACCCCUAAAGAUCCUAUCCUGCGCAGCCUGG
CCUACAGCAACGCCAACAAGGAGUGCCAGAAGCUGCUGCAGGCUCGCGGACAC
ACCAAUAGCCCUCUGGGAGAUAUGCUGCGCGCUUGCCAGACCUGGACCCCCAA
GGACAAGACCAAGGUGCUGGUGGUGCAGCCCAAGAAGCCUCCUCCUAACCAGC
CUUGCUUUCGCUGCGGCAAAGCUGGACACUGGAGCCGCGAUUGCACACAGCCU
CGCCCUCCUCCUGGACCUUGCCCUCUUUGCCAGGAUCCUACCCACUGGAAGCG
CGACUGCCCUCGCCUGAAACCUACCAUCCCUGAACCUGAACCUGAAGAAGACG
CUCUGCUGCUGGAUCUGCCCGCUGAUAUCCCCCACCCCAAGAACAGCAUCGGC
 
GGCGAGGUGUGAUAAGCUGCCUUCUGCGGGGCUUGCCUUCUGGCCAUGCCCUU

CUUCUCUCCCUUGCACCUGUACCUCUUGGUCUUUGAAUAAAGCCUGAGUAGGA

AGGCGGAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAAAA

AAAAAAAAAAAAAAAAAAAAAA
<SEQ ID NO: 1> Amino acid sequence of Gag

MGQIFSRSASPIPRPPRGLAAHHWLNFLQAAYRLEPGPSSYDFHQLKKFLKIALETPV
WICPINYSLLASLLPKGYPGRVNEILHILIQTQAQIPSRPAPPPSSPTHDPPDSDPQIPPP
YVEPTAPQVLPVMHPHGAPPNHRPWQMKDLQAIKQEVSQAAPGSPQFMQTIRLAVQ
QFDPTAKDLQDLLQYLCSSLVASLHHQQLDSLISEAETRGITGYNPLAGPLRVQANN
PQQQGLRREYQQLWLAAFAALPGSAKDPSWASILQGLEEPYHAFVERLNIALDNGLP
EGTPKDPILRSLAYSNANKECQKLLQARGHTNSPLGDMLRACQTWTPKDKTKVLVV
QPKKPPPNQPCFRCGKAGHWSRDCTQPRPPPGPCPLCQDPTHWKRDCPRLKPTIPEPE
PEEDALLLDLPADIPHPKNSIGGEV

### (3) Immunization of mice by intravenous administration and splenocyte preparation

The mouse test conditions have been previously reported (Molecular Therapy Nucleic Acids 2016, 5, e326), to which reference is made. Thus, Lipofectamin RNAiMAX Transfection Reagent (Invitrogen #56532) and mRNA (SEQ ID NO: 33) for the Gag antigen shown in SEQ ID NO: 1 were mixed in Opti-MEM (Gibco #31985062) at a ratio of RNAiMAX : mRNA of 2 : 1 (wt : wt) to prepare lipid-mRNA particles. C57BL/6 mice (female, 10 weeks old, n = 4) received the obtained lipid-mRNA particles by tail vein administration on days 0 and 4 at 0.2 mg/kg and 1 mg/kg mRNA. Opti-MEM was administered by the same method in the negative control group. The mice were euthanized on day 10 and their spleens were removed. The spleens were disrupted on a cell strainer using the plunger of a sterile syringe and were washed with 10 mL RPMI and the cell suspension was collected. The cell suspension was centrifuged, the supernatant was removed, and the cells were hemolyzed with 1 mL of NH4Cl for 2 minutes. The reaction was then stopped with 9 mL RPMI and centrifugation was carried out. The cell pellet was resuspended in 20 mL RPMI with 10% FBS.

### (4) Analysis of the cellular immunity response

The ability to induce a cellular immune response was evaluated using an ELISpot assay. The peptides used to stimulate the cells were designed and pooled using the following method.

Overlapping peptides with a length of 9 amino acids (offset: 1 amino acid) were designed based on the amino acid sequence of Gag. A peptide pool was made by pooling all of the following: 97 peptides for Gag p15, 121 peptides for Gag p19, and 206 peptides for Gag p24.

The ELISpot assay was performed in accordance with the following procedure using ELISpot Flex: IFN-gamma, Mouse-ALP from MABTECH. A Millipore MAIPS4510 assay plate was treated with the coating antibody, and the prepared mouse splenocytes were seeded at 10⁶ cells/well. After stimulation for 24 hours with 0.25 µM peptide pool as described above, the IFN-gamma-producing cells were stained with the detection antibody. After staining, the spots were analyzed using an ImmunoSpot S6 ENTRY Analyzer from Cellular Technology Limited and the number of spots was counted.

The results are reported in Fig. 6 and Fig. 7. Fig. 7 contains a photograph that gives the results of the ELISpot assay; No peptide gives the results for the negative control group (no peptide stimulation) for the respective administrations of 0 mg/kg, 0.2 mg/kg, or 1.0 mg/kg of the lipid-mRNA particles; and Gag Peptide pool gives the results for the respective administrations of 0 mg/kg, 0.2 mg/kg, or 1.0 mg/kg of the lipid-mRNA particles. In Fig. 7C, the first and second rows from the left give the results for No peptide (negative control group, no peptide stimulation) samples 1 to 12 (samples 1 to 4: physiological saline, samples 5 to 8: 0.2 mg/kg, samples 9 to 12: 1 mg/kg); the third and fourth rows from the left give the results for Gag peptide samples 1 to 12 (samples 1 to 4: physiological saline, samples 5 to 8: 0.2 mg/kg, samples 9 to 12: 1 mg/kg); and the fifth and sixth rows from the left give the results for Positive Ctrl samples 1 to 12 (samples 1 to 4: physiological saline, samples 5 to 8: 0.2 mg/kg, samples 9 to 12: 1 mg/kg). In addition, Fig. 6 provides counts of the spots in Fig. 7C for No peptide and Gag peptide.

As shown in Fig. 6 and Fig. 7, spots were formed with the Gag peptide pool (samples 5 to 8 and 9 to 12). These results demonstrated that IFN-γ was produced in mice that had been immunized with lipid-mRNA particles that encapsulated mRNA encoding Gag protein. The amount of IFN-γ production was increased at a higher concentration of administration of the lipid-mRNA particles. The induction of a cellular immune response was suggested by the production of IFN-γ.

The present disclosure has been described in the preceding with reference to embodiments, but the present disclosure is not limited to or by these embodiments. Various modifications and alterations understandable to the individual skilled in the art can be made to the features and details of the present disclosure while within the scope of the present disclosure.

The contents of the patents, patent applications, and documents cited in the present Description are incorporated in the present Description by reference just as if their contents per se had been specifically provided in the present Description.

### <Appendices>

All or part of the embodiments and examples set out in the preceding can be described as in the following appendices, but there is no limitation to or by the following.

### <Pharmaceutical composition comprising HTLV-1 Gag antigen>

### (Appendix 1)

A pharmaceutical composition comprising: human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

### (Appendix 2)

The pharmaceutical composition according to appendix 1, wherein the Gag p15, Gag p19, and/or Gag p24 comprise unprocessed Gag protein.

### (Appendix 3)

The pharmaceutical composition according to appendix 1 or 2, further comprising the Tax protein of HTLV-1 or an immunogenic fragment thereof, and/or the HBZ protein or an immunogenic fragment thereof.

### (Appendix 4)

The pharmaceutical composition according to any of appendices 1 to 3, further comprising an adjuvant.

### (Appendix 5)

The pharmaceutical composition according to any of appendices 1 to 4, for use in the treatment and/or prevention of an HTLV-1-associated disease, or for use in the prevention of the onset of ATL, the aggravation of ATL, or the recurrence of ATL.

### <Nucleic acid-comprising pharmaceutical composition>

### (Appendix 6)

A pharmaceutical composition comprising: a nucleic acid that encodes human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

### (Appendix 7)

The pharmaceutical composition according to appendix 6, comprising a nucleic acid that encodes unprocessed Gag protein as the Gag p15, Gag p19, and/or Gag p24.

### (Appendix 8)

The pharmaceutical composition according to appendix 6 or 7, further comprising a nucleic acid that encodes the Tax protein of HTLV-1 or an immunogenic fragment thereof, and/or the HBZ protein or an immunogenic fragment thereof.

### (Appendix 9)

The pharmaceutical composition according to any of appendices 6 to 8, further comprising an adjuvant.

### (Appendix 10)

The pharmaceutical composition according to any of appendices 6 to 9, for use in the treatment and/or prevention of an HTLV-1-associated disease, or for use in the prevention of the onset of ATL, the aggravation of ATL, or the recurrence of ATL.

### <Vector-containing pharmaceutical composition>

### (Appendix 11)

A pharmaceutical composition comprising: a vector that comprises a nucleic acid that encodes human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

### (Appendix 12)

The pharmaceutical composition according to appendix 11, wherein the vector comprises a nucleic acid that encodes unprocessed Gag protein as the Gag p15, Gag p19, and/or Gag p24.

### (Appendix 13)

The pharmaceutical composition according to appendix 11 or 12, further comprising a vector that comprises a nucleic acid that encodes the Tax protein of HTLV-1 or an immunogenic fragment thereof, and/or the HBZ protein or an immunogenic fragment thereof.

### (Appendix 14)

The pharmaceutical composition according to any of appendices 11 to 13, further comprising an adjuvant.

### (Appendix 15)

The pharmaceutical composition according to any of appendices 11 to 14, for use in the treatment and/or prevention of an HTLV-1-associated disease, or for use in the prevention of the onset of ATL, the aggravation of ATL, or the recurrence of ATL.

### <Prevention method>

### (Appendix 16)

A method for treating and/or preventing an HTLV-1-associated disease, and/or for preventing the onset of ATL, the aggravation of ATL, or the recurrence of ATL, wherein the method uses a pharmaceutical composition according to any of appendices 1 to 15.

### (Appendix 17)

The method of prevention according to appendix 16, that includes an administration step of administering the pharmaceutical composition to a subject.

### <Use>

### (Appendix 18)

The pharmaceutical composition according to any of appendices 1 to 17, for use for treating and/or preventing an HTLV-1-associated disease, or for use for preventing the onset of ATL, the aggravation of ATL, or the recurrence of ATL.

### Industrial Applicability

As has been described in the preceding, the pharmaceutical composition of the present disclosure, for example, can induce an immune response to HTLV-1. Accordingly, the pharmaceutical composition of the present disclosure is expected, for example, to be able to prevent the onset of HTLV-1-associated diseases or the recurrence of HTLV-1-associated diseases. The present disclosure, for example, is very useful in the pharmaceutical sector and so forth.

## Claims

1. A pharmaceutical composition comprising: human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

2. The pharmaceutical composition according to claim 1,wherein the Gag p15, Gag p19, and/or Gag p24 comprise unprocessed Gag protein.

3. The pharmaceutical composition according to claim 1 or 2, further comprising Tax protein of HTLV-1 or an immunogenic fragment thereof, and/or HBZ protein or an immunogenic fragment thereof.

4. The pharmaceutical composition according to claim 1 or 2, further comprising an adjuvant.

5. The pharmaceutical composition according to claim 1 or 2, for use to prevent the onset of ATL, aggravation of ATL, or recurrence of ATL.

6. A pharmaceutical composition comprising: a nucleic acid that encodes human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

7. The pharmaceutical composition according to claim 6, comprising a nucleic acid that encodes unprocessed Gag protein as the Gag p15, Gag p19, and/or Gag p24 .

8. The pharmaceutical composition according to claim 6 or 7, further comprising nucleic acid that encodes Tax protein of HTLV-1 or an immunogenic fragment thereof, and/or HBZ protein or an immunogenic fragment thereof.

9. A pharmaceutical composition comprising: a vector comprising a nucleic acid that encodes human T-cell leukemia virus 1 (HTLV-1) antigenic Gag protein p15 (Gag p15) or an immunogenic fragment thereof, Gag protein p19 (Gag p19) or an immunogenic fragment thereof, and/or Gag protein p24 (Gag p24) or an immunogenic fragment thereof; and a pharmaceutically acceptable carrier.

10. The pharmaceutical composition according to claim 9, wherein the vector comprises a nucleic acid that encodes unprocessed Gag protein as the Gag p15, Gag p19, and/or Gag p24.

11. The pharmaceutical composition according to claim 9 or 10, further comprising a vector comprising a nucleic acid that encodes Tax protein of HTLV-1 or an immunogenic fragment thereof, and/or HBZ protein or an immunogenic fragment thereof.
